# EUROPÄISCHE PATENTSCHRIFT

(11) **EP 2 822 951 B1**
(45) Veröffentlichungstag und Bekanntmachung des Hinweises auf die Patenterteilung: **26.07.2017**
(21) Anmeldenummer: 13712162.0
(22) Anmeldetag: 05.03.2013
(51) Int. Cl.: C07D 487/04, A61K 31/522, A61P 9/00

(54) **SUBSTITUIERTE AZABICYCLEN UND IHRE VERWENDUNG**
SUBSTITUTED AZABICYCLES AND USE THEREOF
AZABICYLES SUBSTITUÉS ET LEUR UTILISATION

(30) Priorität: 06.03.2012 EP 12158166
(43) Veröffentlichungstag der Anmeldung: 14.01.2015
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); VAKALOPOULOS, Alexandros, 40721 Hilden (DE); LANG, Dieter, 42553 Velbert (DE); WUNDER, Frank, 42117 Wuppertal (DE); HÜBSCH, Walter, 42113 Wuppertal (DE); HAHN, Michael, 40764 Langenfeld (DE)
(74) Vertreter: BIP Patents
(86) Internationale Anmeldenummer: PCT/EP2013/054427
(87) Internationale Veröffentlichungsnummer: WO 2013/131923

(56) Entgegenhaltungen:
- WO-A1-2010/065275
- WO-A1-2011/149921
- WO-A1-2011/161099
- WO-A1-2012/004258
- WO-A1-2012/028647
- WO-A1-2012/143510
- WO-A1-2012/152629
- WO-A1-2013/030138
- WO-A1-2013/030288

## Beschreibung

Die vorliegende Anmeldung betrifft neue substituierte Azabicyclen, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.
In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).
Als Stimulatoren der löslichen Guanylatcyclase werden in WO 00/06569 annellierte Pyrazol-Derivate und in WO 01/083490 ein annelliertes Aminopyrimidin-Derivate beschrieben. WO 2010/065275 offenbart Pyrrolopyrimidone als Aktivatoren der löslichen Guanylatcyclase.
Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung. Die vorliegende Erfindung wird durch die Ansprüche definiert.

Gegenstand der Offenbarung sind Verbindungen der allgemeinen Formel (I) 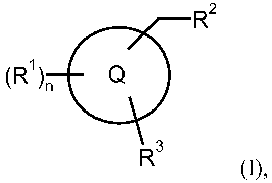 in welcher
der Ring Q für 8- oder 9-gliedriges Heteroaryl steht,
- R¹: für Fluor, Chlor, Cyano, Difluormethyl, Trifluormethyl, (C₁-C₄)-Alkyl, (C₃-C₇)-Cycloalkyl oder (C₁-C₄)-Alkoxy steht,
- n: für eine Zahl 0, 1 oder 2 steht,
- R²: für Trifluormethyl, (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten ausgewählt aus der Gruppe Difluormethyl und Trifluormethyl substituiert ist,
wobei (C₁-C₆)-Alkyl mit 1 bis 3 Substituenten Fluor substituiert sein kann,
wobei (C₃-C₈)-Cycloalkyl mit 1 oder 2 Substituenten unabhängig voneinander ausgewählt aus der Gruppe Fluor, Methyl und Methoxy substituiert sein kann,
wobei Phenyl mit 1 bis 3 Substituenten Fluor substituiert ist,
wobei Phenyl mit 1 oder 2 Substituenten unabhängig voneinander ausgewählt aus der Gruppe Methyl und Methoxy substituiert sein kann,
und
wobei 5- und 6-gliedriges Heteroaryl mit 1 oder 2 Substituenten unabhängig voneinander ausgewählt aus der Gruppe Fluor, Trifluormethyl und Methyl substituiert sein können,
- R³: für eine Gruppe der Formel 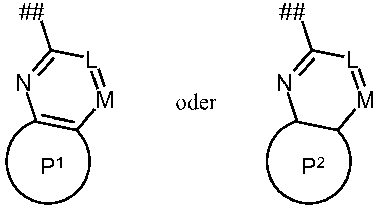 steht, wobei
## für die Anknüpfstelle an den Ring Q steht,
L für CH oder N steht
M für CR⁴ oder N steht,
worin
R⁴ für -R⁵, -OR⁶ oder -NR⁷R⁸ steht,
worin
R⁵ für Wasserstoff, Halogen, Cyano, (C₁-C₆)-Alkyl, (C₂-C₄)-Alkinyl, (C₁-C₄)-Alkylcarbonyl oder (C₁-C₄)-Alkoxycarbonyl steht,
worin (C₁-C₆)-Alkyl und (C₂-C₄)-Alkinyl ihrerseits mit 1 oder 2 Substituenten unabhängig voneinander ausgewählt aus der Gruppe Fluor, Difluormethyl, Trifluormethyl, (C₁-C₆)-Alkyl, (C₃-C₆)-Cycloalkyl, Difluormethoxy, Trifluormethoxy, Oxo -(C=O)ₚ-OR⁹, -C(=o)ₚ-NR⁹R¹⁰, -NR⁹-(C=O)-R¹⁰, -NR⁹-(C=O)-OR¹⁰, -NR'-(C=O)-NR¹⁰R¹¹, -NR⁹-SO₂-R¹⁰, -S(O)_{q}-R¹² und -SO₂-NR⁹R¹⁰ substituiert sein können,
R⁶ für Wasserstoff, (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl, (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₆)-Cycloalkyl, Difluormethoxy, Trifluormethoxy, Oxo, -(C=O)ₚ-OR⁹, -C(=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
R⁷ für Wasserstoff oder (C₁-C₄)-Alkyl steht,
R⁸ für Wasserstoff, (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Difluormethoxy, Trifluormethoxy, Oxo, - (C=O)ₚ-OR⁹, -(C=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹², -SO₂-NR⁹R¹⁰, Phenyl, 4- bis 7-gliedriges Heterocyclyl und 5- oder 6-gliedriges Heteroaryl substituiert sein können,
worin jeweils
p die Zahl 0 oder 1 bedeutet,
q die Zahl 0, 1 oder 2 bedeutet,
R⁹, R¹⁰ und R¹¹ unabhängig voneinander jeweils für Wasserstoff, (C₁-C₆)-Alkyl oder (C₃-C₈)-Cycloalkyl stehen,
worin (C₁-C₆)-Alkyl seinerseits mit 1 oder 2 Substituenten unabhängig voneinander ausgewählt aus der Gruppe Fluor, Difluormethyl, Trifluormethyl, Hydroxy, (C₁-C₆)-Alkoxy, Difluormethoxy, Trifluormethoxy, (C₁-C₆)-Alkoxycarbonyl, Amino, Mono-(C₁-C₆)-alkylamino, Di-(C₁-C₆)-alkylamino und 4- bis 7-gliedriges Heterocyclyl substituiert sein kann, oder
R⁹ und R¹⁰ bilden zusammen mit dem/den Atom/-en, an die sie jeweils gebunden sind, einen 4-bis 7-gliedrigen Heterocyclus,
worin der 4- bis 7-gliedrigen Heterocyclus seinerseits mit 1 oder 2 Substituenten unabhängig voneinander ausgewählt aus der Gruppe Fluor, Cyano, Trifluormethyl, (C₁-C₆)-Alkyl, Hydroxy, Oxo, (C₁-C₆)-Alkoxy, Trifluormethoxy, (C₁-C₆)-Alkoxy-carbonyl, Amino, Mono-(C₁-C₆)-alkyl-amino und Di-(C₁-C₆)-alkylamino substituiert sein kann, oder
R¹⁰ und R¹¹ bilden zusammen mit dem/den Atom/-en, an die sie jeweils gebunden sind, einen 4-bis 7-gliedrigen Heterocyclus,
worin der 4- bis 7-gliedrigen Heterocyclus seinerseits mit 1 oder 2 Substituenten unabhängig voneinander ausgewählt aus der Gruppe Fluor, Cyano, Trifluormethyl, (C₁-C₆)-Alkyl, Hydroxy, Oxo, (C₁-C₆)-Alkoxy, Trifluormethoxy, (C₁-C₆)-Alkoxy-carbonyl, Amino, Mono-(C₁-C₆)-alkylamino und Di-(C₁-C₆)-alkylamino substituiert sein kann, und worin oder worin
R¹² für (C₁-C₆)-Alkyl oder (C₃-C₇)-Cycloalkyl steht,
R⁷ und R⁸ bilden zusammen mit dem Stickstoffatom, an das sie gebunden sind, einen 4- bis 7-gliedrigen Heterocyclus oder ein 5- oder 6-gliedriges Heteroaryl,
worin der 4- bis 7-gliedrigen Heterocyclus und das 5- oder 6-gliedriges Heteroaryl mit 1 bis 3 Substituenten unabhängig voneinander ausgewählt aus der Gruppe Fluor, Cyano, Difluormethyl, Trifluormethyl, (C₁-C₆)-Alkyl, (C₃-C₇)-Cycloalkyl, Hydroxy, Oxo, (C₁-C₆)-Alkoxy, Difluormethoxy, Trifluormethoxy, (C₁-C₆)-Alkoxycarbonyl, Amino, Mono-(C₁-C₆)-alkylamino und Di-(C₁-C₆)-alkylamino substituiert sein können,
und
worin alle zuvor genannten (C₁-C₄)-Alkyl-, (C₁-C₆)-Alkyl-, (C₃-C₈)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
der Ring P¹ für 5- bis 7-gliedriges Heterocyclyl, Phenyl oder 5- oder 6-gliedriges Heteroaryl steht,
worin 5- bis 7-gliedriges Heterocyclyl, Phenyl und 5- oder 6-gliedriges Heteroaryl mit 1 bis 3 Substituenten unabhängig voneinander ausgewählt aus der Gruppe Halogen, Cyano, Difluormethyl, Trifluormethyl, Trideuteromethyl, (C₁-C₆)-Alkyl, (C₂-C₆)-Alkenyl, (C₃-C₇)-Cycloalkyl, Oxo, Difluormethoxy, Trifluormethoxy, Thiooxo und einer Gruppe der Formel -M-R¹³ substituiert sein können,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Difluormethoxy und Trifluormethoxy substituiert sein kann,

der Ring P² für 5-gliedriges Heteroaryl steht,
worin 5-gliedriges Heteroaryl mit 1 bis 3 Substituenten unabhängig voneinander ausgewählt aus der Gruppe Halogen, Cyano, Difluormethyl, Trifluormethyl, Trideuteromethyl, (C₁-C₆)-Alkyl, (C₂-C₆)-Alkenyl, (C₃-C₇)-Cycloalkyl, Oxo, Difluormethoxy, Trifluormethoxy, Thiooxo und einer Gruppe der Formel -M-R¹³ substituiert sein können,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Difluormethoxy und Trifluormethoxy substituiert sein kann,
und worin jeweils
M für eine Bindung oder (C₁-C₄)-Alkandiyl steht,
R¹³ für -(C=O)ᵣ-OR¹⁴, -(C=O)ᵣ-NR¹⁴R¹⁵, -C(=S)-NR¹⁴R¹⁵, - NR¹⁴-(C=O)-R¹⁵, -NR¹⁴-(C=O)-OR¹⁷, -NR¹⁴-(C=O)-NR¹⁵R¹⁶, -NR¹⁴-SO₂-NR¹⁵R¹⁶, -NR¹⁴-SO₂-R¹⁷, -S(O)ₛ-R¹⁷, -SO₂-NR¹⁴R¹⁵, 4- bis 7-gliedriges Heterocyclyl, Phenyl, Benzyl oder 5- oder 6-gliedriges Heteroaryl steht, worin
r die Zahl 0 oder 1 bedeutet,
s die Zahl 0, 1 oder 2 bedeutet,
R¹⁴, R¹⁵ und R¹⁶ unabhängig voneinander jeweils für Wasserstoff, (C₁-C₆)-Alkyl, (C₃-C₈)-Cycloalkyl, 4- bis 7-gliedriges Heterocyclyl, Phenyl, Benzyl oder 5- oder 6-gliedriges Heteroaryl stehen, oder
R¹⁴ und R¹⁵ bilden zusammen mit dem/den Atom/-en, an die sie jeweils gebunden sind, einen 4-bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
R¹⁴ und R¹⁵ bilden zusammen mit dem/den Atom/-en, an die sie jeweils gebunden sind, einen 4-bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹⁷ für (C₁-C₆)-Alkyl oder (C₃-C₇)-Cycloalkyl steht, oder
R¹⁴ und R¹⁷ bilden zusammen mit dem/den Atom/-en, an die sie jeweils gebunden sind, einen 4-bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, und
worin 4- bis 7-gliedriges Heterocyclyl, Phenyl, Benzyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen, sofern nicht anders angegeben, jeweils unabhängig voneinander weiterhin mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
sowie ihre *N-*Oxide, Salze, Solvate, Salze der *N-*Oxide und Solvate der *N-*Oxide und Salze,
mit Ausnahme der Verbindungen:
2-[1-(2-Fluorbenzyl)-1H-pyrazolo[3,4-b]pyridin-3-yl]-8-methyl-9H-purin,
2-[1-(2-Fluorbenzyl)-1H-pyrazolo[3,4-b]pyridin-3-yl]-8-methyl-9H-purin-6-amin,
N-Butyl-2-[1-(2-fluorbenzyl)-1H-pyrazolo[3,4-b]pyridin-3-yl]-8-methyl-9H-purin-6-amin.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Offenbarung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Offenbarung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.
Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.
5- bis 7-gliedrigen gesättigter oder teilweise ungesättigter Carbocyclus steht in Rahmen der Erfindung für einen gesättigten oder teilweise ungesättigten cyclischen Alkylrest mit der jeweils angegebenen Anzahl an Kohlenstoffatomen. Beispielhaft und vorzugsweise seien genannt: Cyclopentyl, Cyclohexyl, Cycloheptyl, Cyclopentenyl, Cyclohexenyl und Cycloheptenyl.
Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-diyl, Propan-1,2-diyl, Propan-2,2-diyl, Butan-1,4-diyl, Butan-1,2-diyl, Butan-1,3-diyl und Butan-2,3-diyl. Bevorzugt sind: Methylen, Ethan-1,2-diyl, Propan-1,3-diyl und Butan-1,4-diyl.
Alkenyl steht im Rahmen der Erfindung für einen linearen oder verzweigten Alkenylrest mit 2 bis 6 bzw. 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkoxycarbonyl steht im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
5- bis 7-gliedrigen gesättigter oder teilweise ungesättigter Heterocyclus steht im Rahmen der Erfindung für einen gesättigten oder teilweise ungesättigten Heterocyclus mit insgesamt 5 bis 7 Ringatomen, der ein Ring-Heteroatome aus der Reihe N, O, S, SO und/oder SO₂ enthält. Beispielhaft seien genannt: Pyrrolidinyl, Tetrahydrofuranyl, Piperidinyl, Tetrahydropyranyl, Dihydropyrrolyl, Dihydropyridyl.
Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.
5- oder 6-gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.
8- oder 9-gliedrige Heteroaryl steht im Rahmen der Erfindung für einen bicyclischen aromatischen oder teilweise ungesättigten Heterocyclus mit insgesamt 8 oder 9 Ringatomen, der mindestens zwei Stickstoffatome und bis zu zwei weitere, gleiche oder verschiedene Ring-Heteroatome aus der Reihe N, O und/oder S enthält. Beispielhaft seien genannt: Dihydrothienopyrazolyl, Thienopyrazolyl, Pyrazolopyrazolyl, Imidazothiazolyl, Tetrahydrocyclopentapyrazolyl, Dihydrocyclopentapyrazolyl, Tetrahydroindazolyl, Dihydroindazolyl, Indazolyl, Pyrazolopyridinyl, Tetrahydropyrazolopyridinyl, Pyrazolopyrimidinyl, Imidazopyridinyl und Imidazopyridazinyl.
Halogen steht im Rahmen der Erfindung für Fluor, Chlor, Brom und Iod. Bevorzugt sind Fluor und Chlor.

Eine Oxo-Gruppe steht im Rahmen der Erfindung für ein Sauerstoffatom, das über eine Doppelbindung an ein Kohlenstoffatom gebunden ist.

Eine Thiooxo-Gruppe steht im Rahmen der Erfindung für ein Schwefelatom, das über eine Doppelbindung an ein Kohlenstoffatom gebunden ist.

In der Formel der Gruppe, für die Q bzw. R³ stehen kann, steht der Endpunkt der Linie, an dem das Zeichen *, ** bzw. ## steht, nicht für ein Kohlenstoffatom beziehungsweise eine CH₂-Gruppe, sondern ist Bestandteil der Bindung zu dem jeweils bezeichneten Atom, an das L bzw. R³ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Offenbarungsgegenstand sind Verbindungen der Formel (I), in welcher der Ring Q für eine Gruppe der Formel 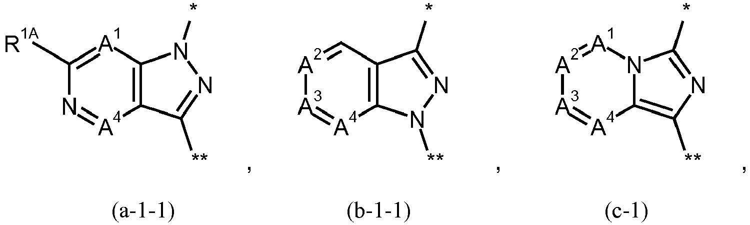 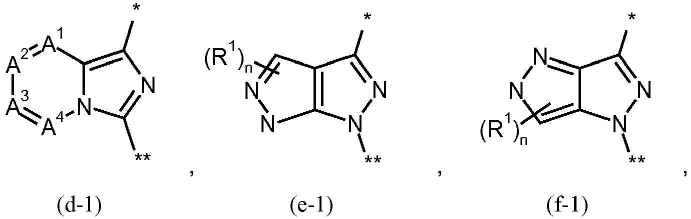 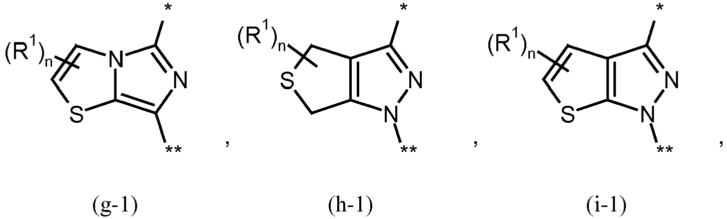 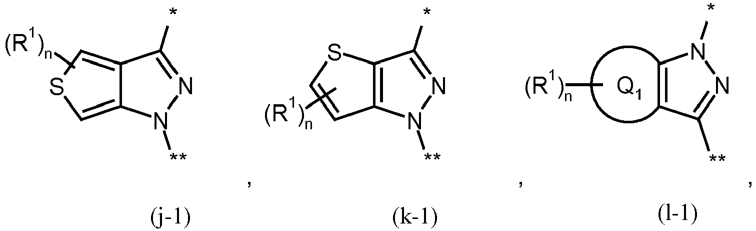 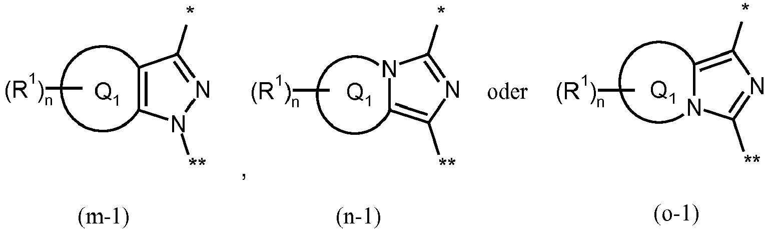 steht, wobei
- *: für die Anknüpfungsstelle an -CH₂-R² steht,
- **: für die Anknüpfungsstelle an den R³ steht,
- der Ring Q₁: zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R¹: für Fluor, Chlor, Methyl, Hydroxy oder Oxo steht,
- R^{1A}: für Wasserstoff oder Methyl steht,
- n: für eine Zahl 0, 1 oder 2 steht,
- A¹, A², A³ und A⁴: unabhängig voneinander jeweils für N, CH oder CR¹ stehen,
mit der Maßgabe, dass maximal zwei der Gruppen A¹, A², A³ und A⁴ für N stehen,
- R²: für Trifluormethyl, 2,2,2-Trifluorethyl, 3,3,3-Trifluorprop-1-yl, 2,2,3,3-Tetrafluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
- R³: für eine Gruppe der Formel 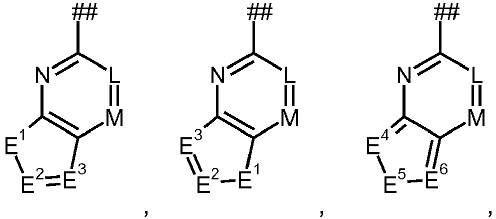 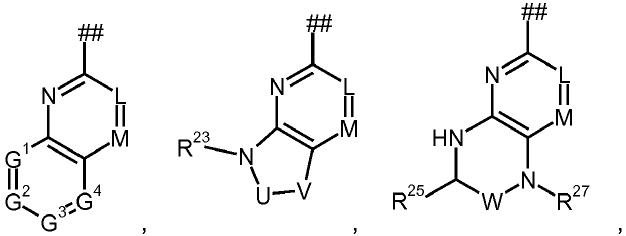 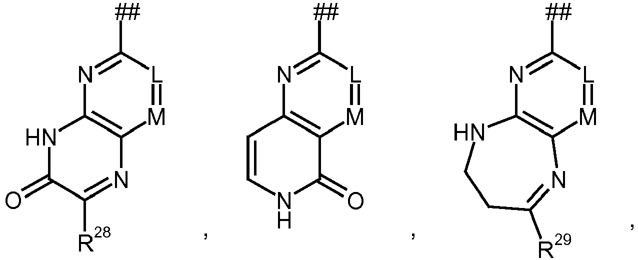 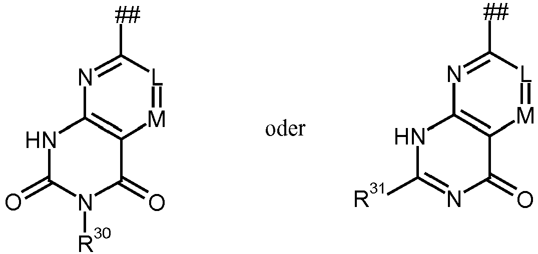 steht, wobei
## für die Anknüpfstelle an den Ring Q steht,
L für CH oder N steht
M für CR⁴ oder N steht,
worin
- R⁴: für -R⁵, -OR⁶ oder -NR⁷R⁸ steht,
worin
R⁵ für Wasserstoff, Chlor, Cyano, (C₁-C₄)-Alkyl oder (C₂-C₄)-Alkinyl steht,
worin (C₁-C₄)-Alkyl und (C₂-C₄)-Alkinyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹ und -C(=O)ₚ-NR⁹R¹⁰ substituiert sein können,
worin
p die Zahl 0 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁶ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, und -NR⁹-(C=O)-R¹⁰ substituiert sein kann,
und
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und - (C=O)ₚ-NR⁹R¹⁰ substituiert sein können,
worin jeweils
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
- R⁷: für Wasserstoff, Methyl oder Ethyl steht,
- R⁸: für Wasserstoff, (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl, Cyclopropyl, Cyclobutyl und Cyclopentyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann und
worin Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können,
und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
- p: die Zahl 0 oder 1 bedeutet,
- R⁹ und R¹⁰: unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
oder
- R⁹ und R¹⁰: bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
oder
- R⁷ und R⁸: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl-oder Triazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein kann,
- E¹: für O, S oder NR¹⁸ steht,
worin
R¹⁸ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
E² für N steht,
E³ für N oder CR¹⁹ steht,
worin
R¹⁹ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁴ und E⁶: unabhängig voneinander jeweils für N oder CR²⁰ stehen,
worin
R²⁰ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁵: für NR²¹ steht,
worin
R²¹ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
- G¹, G², G³ und G⁴: unabhängig voneinander jeweils für N oder CR²² stehen,
worin
R²² für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht, worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht,
und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
mit der Maßgabe, dass maximal zwei der Gruppen G¹, G², G³ und G⁴ für Stickstoff stehen,
und
mit der Maßgabe, dass mindestens eine der Gruppen G¹, G², G³ und G⁴ für CH steht,
- U: für C=O, C=S oder SO₂ steht,
- V: für O oder NR²⁴ steht,
worin
R²⁴ für Wasserstoff, Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
- W: für N oder CR²⁵ steht,
worin
R²⁵ für Wasserstoff oder Oxo steht,
- R²³: für Wasserstoff, Trideuteromethyl, (C₁-C₆)-Alkyl oder (C₂-C₆)-Alkenyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R²⁵: für Wasserstoff oder Oxo steht,
- R²⁷: für Wasserstoff, (C₁-C₅)-Alkoxycarbonyl oder Aminosulfonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
- R²⁸: für Wasserstoff, Trifluormethyl, (C₁-C₄)-Alkyl, (C₃-C₆)-Cycloalkyl und Phenyl steht,
worin Phenyl mit 1 oder 2 Substituenten unabhängig voneinander ausgewählt aus der Gruppe Fluor, Chlor, Trifluormethyl, (C₁-C₄)-Alkyl, Methoxy und Ethoxy substituiert sein kann,
- R²⁹: für Wasserstoff, Trifluormethyl, (C₁-C₄)-Alkyl, Cyclopropyl, Cyclobutyl und Phenyl steht,
worin Phenyl mit 1 oder 2 Substituenten unabhängig voneinander ausgewählt aus der Gruppe Fluor, Chlor, Trifluormethyl, Methyl, Ethyl, Methoxy und Ethoxy substituiert sein kann,
- R³⁰: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R³¹: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
sowie ihre Salze, Solvate und Solvate der Salze.

Weiterer Offenbarungsgegenstand sind Verbindungen der Formel (I), in welcher
der Ring Q für eine Gruppe der Formel 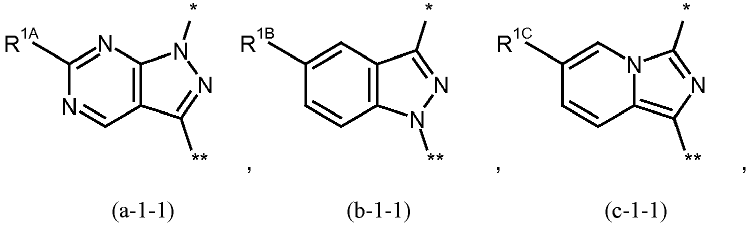 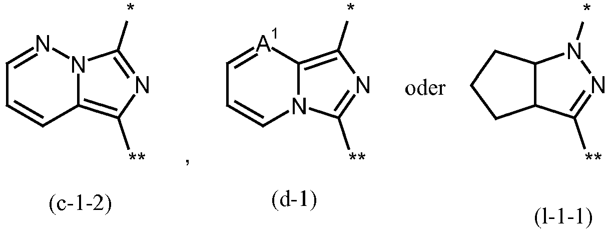 steht, wobei
- *: für die Anknüpfungsstelle an -CH₂-R² steht,
- **: für die Anknüpfungsstelle an den R³ steht,
- R^{1A}: für Wasserstoff oder Methyl steht,
- R^{1B}: für Wasserstoff oder Fluor steht,
- R^{1C}: für Wasserstoff oder Chlor steht,
- A¹: für N oder CH steht,
- R²: für 3,3,3-Trifluorprop-1-yl, 2,2,3,3-Tetrefluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
- R³: für eine Gruppe der Formel 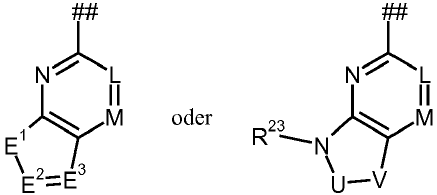 steht, wobei
## für die Anknüpfstelle an den Ring Q steht,
L für N steht
M für CR⁴ steht,
worin
R⁴ für -R⁵, -OR⁶ oder -NR⁷R⁸ steht,
worin
R⁵ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Difluormethyl, Trifluormethyl, Cyclopropyl, Cyclobutyl, -(C=O)ₚ-OR⁹ und -C(=O)ₚ-NR⁹R¹⁰ substituiert sein kann,
worin
p die Zahl 0 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
R⁶ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
p die Zahl 0 oder 1 bedeutet,
R⁹ und R¹⁰ unabhängig voneinander jeweils für Wasserstoff oder Methyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann, und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl und Cyclopentyl substituiert sein kann,
- R⁷: für Wasserstoff, Methyl oder Ethyl steht,
- R⁸: für Wasserstoff, (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
p die Zahl 0 oder 1 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo, Difluormethoxy, Trifluormethoxy, Methoxy und Ethoxy substituiert sein können, und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl und Trifluormethoxy substituiert sein können,
- E¹: für NR¹⁸ steht,
worin
R¹⁸ für Wasserstoff steht,
- E²: für N steht,
- E³: für N oder CR¹⁹ steht,
worin
R¹⁹ für Wasserstoff, Fluor, Difluormethyl, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 0 oder 1 bedeutet,
R¹⁴ und R¹⁵ unabhängig voneinander jeweils für Wasserstoff, Methyl, Ethyl, Cyclopropyl, Cyclobutyl oder Phenyl stehen,
worin Methyl und Ethyl weiterhin mit 1 oder 2 Substituenten unabhängig voneinander ausgewählt aus der Gruppe Fluor, Difluormethyl, Trifluormethyl, Cyclopropyl, Cyclobutyl, Hydroxy, Trifluormethoxy und Methoxy substituiert sein können, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmethyl und Cyclobutylmethyl substituiert sein können,
- U: für C=O steht,
- V: für NR²⁴ steht,
worin
R²⁴ für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-4-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Cyclopropyl und Cyclobutyl substituiert sein können,
- R²³: für Wasserstoff steht,
sowie ihre Salze, Solvate und Solvate der Salze.

Weiterer Offenbarungsgegenstand sind Verbindungen der Formel (I), in welcher
der Ring Q für eine Gruppe der Formel 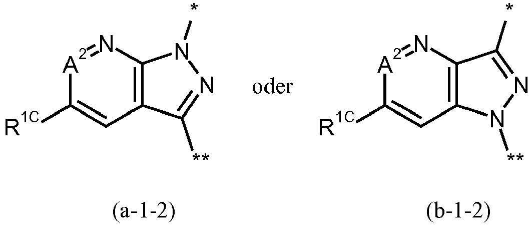 steht, wobei
- *: für die Anknüpfungsstelle an -CH₂-R² steht,
- **: für die Anknüpfungsstelle an den R³ steht,
- R^{1C}: für Wasserstoff oder Fluor steht,
- A²: für N oder CH stehen,
- R²: für Cyclopropyl, Cyclobutyl, Cyclopentyl, Cyclohexyl, Pyridyl, Pyrimidinyl, Pyrazinyl oder Pyridazinyl steht,
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
- R³: für eine Gruppe der Formel 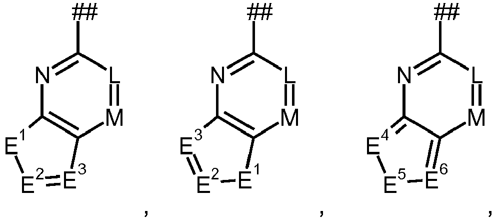 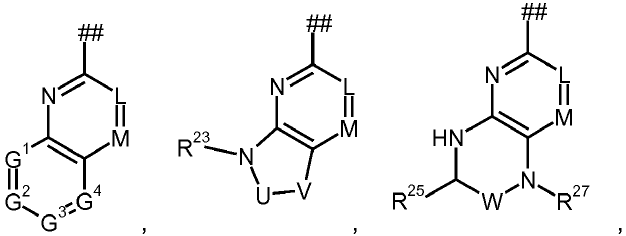 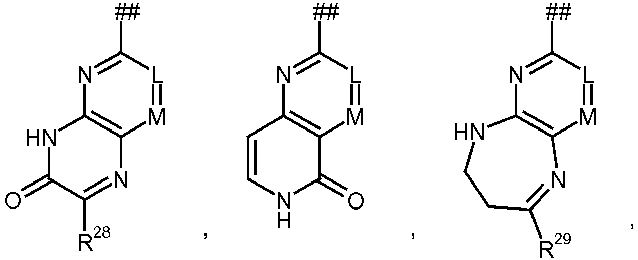 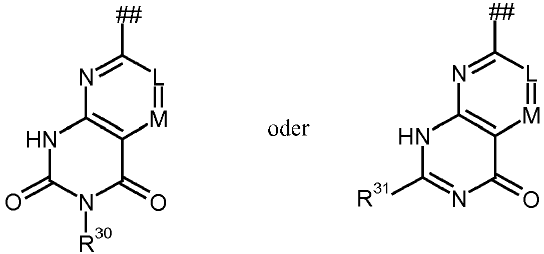 steht, wobei
## für die Anknüpfstelle an den Ring Q steht,
L für CH oder N steht
M für CR⁴ oder N steht,
worin
R⁴ für -R⁵, -OR⁶ oder -NR⁷R⁸ steht,
worin
R⁵ für Wasserstoff, Chlor, Cyano, (C₁-C₄)-Alkyl oder (C₂-C₄)-Alkinyl steht,
worin (C₁-C₄)-Alkyl und (C₂-C₄)-Alkinyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹ und -C(=O)ₚ-NR⁹R¹⁰ substituiert sein können,
worin
p die Zahl 0 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁶ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR-, -(C=O)ₚ-NR⁹R¹⁰, und -NR⁹-(C=O)-R¹⁰ substituiert sein kann,
und
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und - (C=O)ₚ-NR⁹R¹⁰ substituiert sein können, worin
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
- R⁷: für Wasserstoff, Methyl oder Ethyl steht,
- R⁸: für Wasserstoff, (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht, worin (C₁-C₆)-Alkyl, Cyclopropyl, Cyclobutyl und Cyclopentyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann und
worin Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können, und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann, oder
R⁷ und R⁸ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl-oder Triazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- E¹: für O, S oder NR¹⁸ steht,
worin
R¹⁸ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
- E²: für N steht,
- E³: für N oder CR¹⁹ steht,
worin
R¹⁹ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁴ und E⁶: unabhängig voneinander jeweils für N oder CR²⁰ stehen,
worin
R²⁰ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁵: für NR²¹ steht,
worin
R²¹ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
- G¹, G², G³ und G⁴: unabhängig voneinander jeweils für N oder CR²² stehen,
worin
R²² für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
mit der Maßgabe, dass maximal zwei der Gruppen G¹, G², G³ und G⁴ für Stickstoff stehen,
und
mit der Maßgabe, dass mindestens eine der Gruppen G¹, G², G³ und G⁴ für CH steht,
- U: für C=O, C=S oder SO₂ steht,
- V: für O oder NR²⁴ steht,
worin
R²⁴ für Wasserstoff, Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
- W: für N oder CR²⁵ steht,
worin
R²⁵ für Wasserstoff oder Oxo steht,
- R²³: für Wasserstoff, Trideuteromethyl, (C₁-C₆)-Alkyl oder (C₂-C₆)-Alkenyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R²⁵: für Wasserstoff oder Oxo steht,
- R²⁷: für Wasserstoff, (C₁-C₃)-Alkoxycarbonyl oder Aminosulfonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
- R²⁸: für Wasserstoff, Trifluormethyl, (C₁-C₄)-Alkyl, (C₃-C₆)-Cycloalkyl und Phenyl steht,
worin Phenyl mit 1 oder 2 Substituenten unabhängig voneinander ausgewählt aus der Gruppe Fluor, Chlor, Trifluormethyl, (C₁-C₄)-Alkyl, Methoxy und Ethoxy substituiert sein kann,
- R²⁹: für Wasserstoff, Trifluormethyl, (C₁-C₄)-Alkyl, Cyclopropyl, Cyclobutyl und Phenyl steht,
worin Phenyl mit 1 oder 2 Substituenten unabhängig voneinander ausgewählt aus der Gruppe Fluor, Chlor, Trifluormethyl, Methyl, Ethyl, Methoxy und Ethoxy substituiert sein kann,
- R³⁰: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R³¹: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
sowie ihre Salze, Solvate und Solvate der Salze.

Weiterer Offenbarungsgegenstand sind Verbindungen der Formel (I), in welcher
der Ring Q für eine Gruppe der Formel 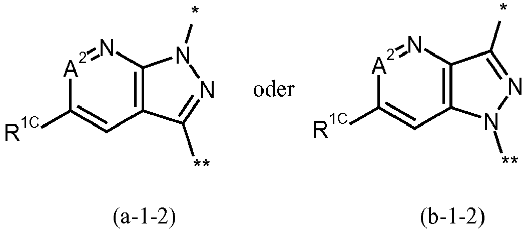 steht, wobei
- *: für die Anknüpfungsstelle an -CH₂-R² steht,
- **: für die Anknüpfungsstelle an den R³ steht,
- R^{1C}: für Wasserstoff oder Fluor steht,
- A²: für N oder CH steht,
- R²: für Trifluormethyl, 2,2,2-Trifluorethyl, 3,3,3-Trifluorprop-1-yl, 2,2,3,3-Tetrafluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
- R³: für eine Gruppe der Formel 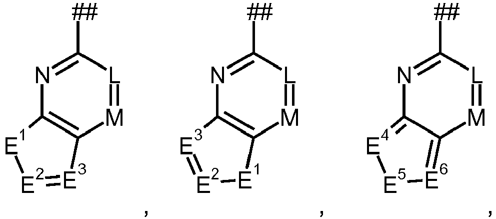 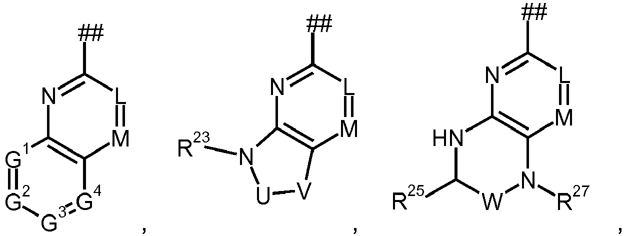 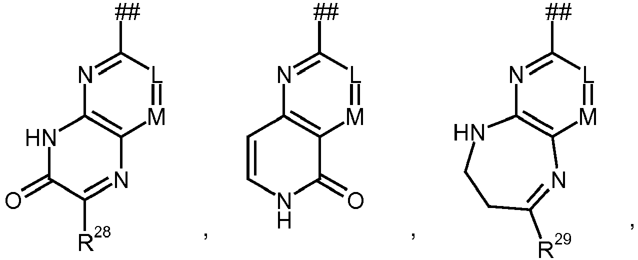 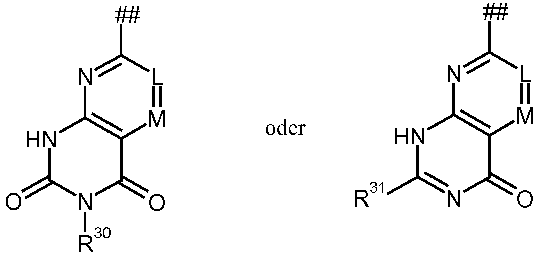 steht, wobei
## für die Anknüpfstelle an den Ring Q steht,
L für CH oder N steht
M für CR⁴ oder N steht,
worin
R⁴ für -R⁵, -OR⁶ oder -NR⁷R⁸ steht,
worin
R⁵ für Trifluormethyl oder (C₁-C₄)-Alkyl steht,
worin (C₁-C₄)-Alkyl ihrerseits mit 1 oder 2 Substituenten unabhängig voneinander ausgewählt aus der Gruppe Difluormethyl, Trifluormethyl, Cyclopropyl, Cyclobutyl, Cyclopentyl, Difluormethoxy, Trifluormethoxy, -(C=O)ₚ-OR⁹ und -C(=O)ₚ-NR⁹R¹⁰ substituiert sein kann,
worin
p die Zahl 0 bedeutet,
R⁹ und R¹⁰ unabhängig voneinander jeweils für Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁶ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Difluormethyl, Trifluormethyl, Cyclopropyl, Cyclobutyl, Cyclopentyl, Difluormethoxy, Trifluormethoxy, (C₁-C₄)-Alkoxy, -(C=O)-OR⁹, -(C=O)-NR⁹R¹⁰, und -NR⁹-(C=O)-R¹⁰ substituiert ist,
worin
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann, und
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und - (C=O)ₚ-NR⁹R¹⁰ substituiert sein können,
worin jeweils
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁷ für Wasserstoff, Methyl oder Ethyl steht,
R⁸ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl, Cyclopropyl, Cyclobutyl und Cyclopentyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, (C₁-C₄)-Alkoxy, -(C=O)-OR⁹, -(C=O)-NR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann und
worin Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können, und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
p die Zahl 0 oder 1 bedeutet,
R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann, oder
- R⁷ und R⁸: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl-oder Triazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- E¹: für O, S oder NR¹⁸ steht,
worin
R¹⁸ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
E² für N steht,
E³ für N oder CR¹⁹ steht,
worin
R¹⁹ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁴ und: E⁶ unabhängig voneinander jeweils für N oder CR²⁰ stehen,
worin
R²⁰ für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
- E⁵: für NR²¹ steht,
worin
R²¹ für Wasserstoff oder (C₁-C₄)-Alkyl steht,
worin (C₁-C₄)-Alkyl seinerseits mit 1 oder 2 Substituenten unabhängig voneinander ausgewählt aus der Gruppe Fluor, Trifluormethyl, Hydroxy, Methoxy und Ethoxy substituiert sein kann,
- G¹, G²,: G³ und G⁴ unabhängig voneinander jeweils für N oder CR²² stehen,
worin
R²² für Wasserstoff, Fluor, Difluormethyl, Trifluormethyl, (C₁-C₄)-Alkyl, Cyclopropyl, Cyclobutyl, Cyclopentyl, oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Difluormethoxy und Trifluormethoxy substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-OR¹⁷, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können,
R¹⁷ für Methyl, Ethyl, iso-Propyl, Cyclopropyl, Cyclobutyl, Cyclopentyl steht, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
mit der Maßgabe, dass maximal zwei der Gruppen G¹, G², G³ und G⁴ für Stickstoff stehen, und
mit der Maßgabe, dass mindestens eine der Gruppen G¹, G², G³ und G⁴ für CH steht,
- U: für C=O, C=S oder SO₂ steht,
- V: für O oder NR²⁴ steht,
worin
R²⁴ für Wasserstoff, Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Methoxy, Ethoxy, Methylsulfonyl, Ethylsulfonyl, Azetidinyl, Oxetanyl, Pyrrolidinyl, Piperidinyl und Morpholinyl substituiert sein kann,
worin Azetidinyl, Oxetanyl, Pyrrolidinyl, Piperidinyl und Morpholinyl wiederum ihrerseits mit 1 oder 2 Substituenten unabhängig voneinander ausgewählt aus der Gruppe Fluor, Trifluormethyl, 2,2-Difluorethyl, 2,2,2-Trifluorethyl, Methyl und Ethyl substituiert sein können,
worin Azetidinyl, Pyrrolidinyl und Piperidinyl ihrerseits mit 1 oder 2 Substituenten unabhängig voneinander ausgewählt aus der Gruppe Fluor, Trifluormethyl, Methyl, Ethyl, Cyclopropyl und Cyclobutyl substituiert sein können,
worin Methyl und Ethyl wiederum ihrerseits mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein können,
und
worin Benzyl seinerseits mit 1 oder 2 Substituenten Fluor, Chlor, Trifluormethyl, Methyl, Ethyl, Methylsulfonyl und Ethylsulfonyl substituiert sein kann,
- W: für N oder CR²⁵ steht,
worin
R²⁵ für Wasserstoff oder Oxo steht,
- R²³: für Wasserstoff, Trideuteromethyl, (C₁-C₆)-Alkyl oder (C₂-C₆)-Alkenyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R²⁵: für Wasserstoff oder Oxo steht,
- R²⁷: für Wasserstoff, (C₁-C₃)-Alkoxycarbonyl oder Aminosulfonyl steht,
worin (C₁-C₃)-Alkoxycarbonyl mit 1 oder 2 Substituenten unabhängig voneinander ausgewählt aus der Gruppe Fluor, Trifluormethyl, Hydroxy, Methoxy und Ethoxy substituiert sein können,
- R²⁸: für Wasserstoff, Trifluormethyl, (C₁-C₄)-Alkyl, (C₃-C₆)-Cycloalkyl und Phenyl steht,
worin Phenyl mit 1 oder 2 Substituenten unabhängig voneinander ausgewählt aus der Gruppe Fluor, Chlor, Trifluormethyl, (C₁-C₄)-Alkyl, Methoxy und Ethoxy substituiert sein kann,
- R²⁹: für Wasserstoff, Trifluormethyl, (C₁-C₄)-Alkyl, Cyclopropyl, Cyclobutyl und Phenyl steht,
worin Phenyl mit 1 oder 2 Substituenten unabhängig voneinander ausgewählt aus der Gruppe Fluor, Chlor, Trifluormethyl, Methyl, Ethyl, Methoxy und Ethoxy substituiert sein kann,
- R³⁰: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
- R³¹: für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy und Ethoxy substituiert sein kann,
sowie ihre Salze, Solvate und Solvate der Salze,
mit Ausnahme der Verbindungen:
2-[1-(2-Fluorbenzyl)-1H-pyrazolo[3,4-b]pyridin-3-yl]-8-methyl-9H-purin,
2-[1-(2-Fluorbenzyl)-1H-pyrazolo[3,4-b]pyridin-3-yl]-8-methyl-9H-purin-6-amin,
N-Butyl-2-[1-(2-fluorbenzyl)-1H-pyrazolo[3,4-b]pyridin-3-yl]-8-methyl-9H-purin-6-amin.

Weiterer Offenbarungsgegenstand sind Verbindungen der Formel (I), in welcher
der Ring Q für eine Gruppe der Formel 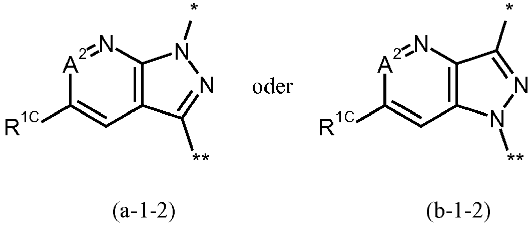 steht, wobei
- *: für die Anknüpfungsstelle an -CH₂-R² steht,
- **: für die Anknüpfungsstelle an den R³ steht,
- R^{1C}: für Wasserstoff, Fluor oder Methyl steht,
- A²: für CH steht,
- R²: für Pyridyl oder Pyrimidinyl steht, wobei Pyridyl und Pyrimidinyl mit 1 Substituenten Fluor substituiert sein können,
R³ für eine Gruppe der Formel 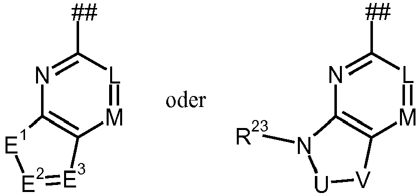 steht, wobei
## für die Anknüpfstelle an den Ring Q steht,
L für N steht
M für CR⁴ steht,
worin
R⁴ für -R⁵, -OR⁶ oder -NR⁷R⁸ steht,
worin
R⁵ für Wasserstoff, Trifluormethyl oder (C₁-C₄)-Alkyl steht,
worin (C₁-C₄)-Alkyl seinerseits mit 1 oder 2 Substituenten unabhängig voneinander ausgewählt aus der Gruppe Fluor, Difluormethyl, Trifluormethyl, Cyclopropyl, Cyclobutyl, -(C=O)ₚ-OR⁹ und -C(=O)p-NR⁹R¹⁰ substituiert sein kann,
worin
p die Zahl 0 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
R⁶ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
p die Zahl 0 oder 1 bedeutet,
R⁹ und R¹⁰ unabhängig voneinander jeweils für Wasserstoff oder Methyl stehen, oder
R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann, und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl und Cyclopentyl substituiert sein kann,
R⁷ für Wasserstoff, Methyl oder Ethyl steht,
R⁸ für Wasserstoff, (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert sind,
worin
p die Zahl 0 oder 1 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo, Difluormethoxy, Trifluormethoxy, Methoxy und Ethoxy substituiert sein können, und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl und Trifluormethoxy substituiert sein können,
- E¹: für NR¹⁸ steht,
worin
R¹⁸ für Wasserstoff steht,
- E²: für N steht,
- E³: für N oder CR¹⁹ steht,
worin
R¹⁹ für Wasserstoff, Fluor, Difluormethyl, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 0 oder 1 bedeutet,
R¹⁴ und R¹⁵ unabhängig voneinander jeweils für Wasserstoff, Methyl, Ethyl, Cyclopropyl, Cyclobutyl oder Phenyl stehen,
worin Methyl und Ethyl weiterhin mit 1 oder 2 Substituenten unabhängig voneinander ausgewählt aus der Gruppe Fluor, Difluormethyl, Trifluormethyl, Cyclopropyl, Cyclobutyl, Hydroxy, Trifluormethoxy und Methoxy substituiert sein können, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmethyl und Cyclobutylmethyl substituiert sein können,
- U: für C=O steht,
- V: für NR²⁴ steht,
worin
R²⁴ für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-4-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Cyclopropyl und Cyclobutyl substituiert sein können,
R²³ für Wasserstoff steht,
sowie ihre Salze, Solvate und Solvate der Salze.

Weiterer Offenbarungsgegenstand sind Verbindungen der Formel (I), in welcher
der Ring Q für eine Gruppe der Formel 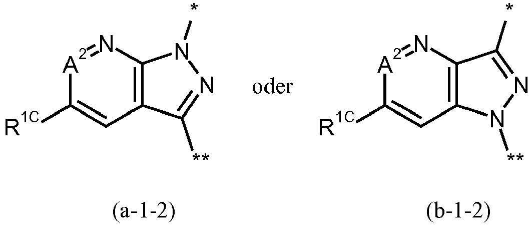 steht, wobei
- *: für die Anknüpfungsstelle an -CH₂-R² steht,
- **: für die Anknüpfungsstelle an den R³ steht,
- R^{1C}: für Wasserstoff oder Fluor steht,
- A²: für CH steht,
- R²: für 3-Fluorpyrid-2-yl steht,
- R³: für eine Gruppe der Formel 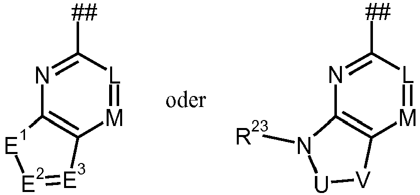 steht, wobei
## für die Anknüpfstelle an den Ring Q steht,
L für N steht
M für CR⁴ steht,
worin
R⁴ für -R⁵ oder -NR⁷R⁸ steht,
worin
R⁵ für Wasserstoff steht,
R⁷ für Wasserstoff steht,
R⁸ fürWasserstoff oder (C₁-C₆)-Alkyl steht,
worin (C₁-C₆)-Alkyl mit 1 bis 3 Substituenten unabhängig voneinander ausgewählt aus der Gruppe Fluor, Trifluormethyl, Cyclopropyl, Cyclobutyl, -(C=O)ₚ-OR⁹ und -(C=O)ₚ-NR⁹R¹⁰ substituiert ist,
worin
p die Zahl 0 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
E¹ für NR¹⁸ steht,
worin
R¹⁸ für Wasserstoff steht,
- E²: für N steht,
- E³: für CR¹⁹ steht,
worin
R¹⁹ für Wasserstoff, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung steht,
R¹³ für -(C=O)ᵣ-OR¹⁴ oder -(C=O)ᵣ-NR¹⁴R¹⁵ steht,
worin
r die Zahl 0 bedeutet,
R¹⁴ und R¹⁵ unabhängig voneinander jeweils für Wasserstoff, Methyl, Ethyl, Cyclopropyl oder Cyclobutyl stehen,
- U: für C=O steht,
- V: für NR²⁴ steht,
worin
R²⁴ für (C₁-C₆)-Alkyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl und Hydroxy substituiert sein kann,
- R²³: für Wasserstoff steht,
sowie ihre Salze, Solvate und Solvate der Salze.

Weiterer Offenbarungsgegenstand sind Verbindungen der Formel (I), in welcher
der Ring Q für eine Gruppe der Formel 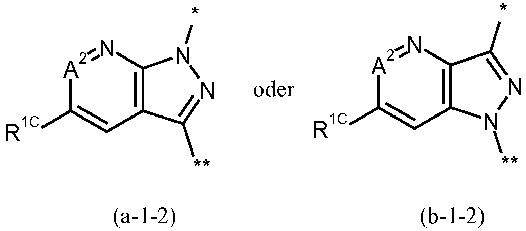 steht, wobei
- *: für die Anknüpfungsstelle an -CH₂-R² steht,
- **: für die Anknüpfungsstelle an den R³ steht,
R^{1C} für Wasserstoff oder Fluor steht,
A² für CH steht,
R² für 3,3,3-Trifluorprop-1-yl, 2,2,3,3-Tetrafluorprop-1-yl, 2,2,3,3,3-Pentafluorprop-1-yl, Phenyl, Pyridyl oder Pyrimidinyl steht,
wobei Phenyl mit 1 bis 3 Substituenten Fluor substituiert ist,
und
wobei Pyridyl und Pyrimidinyl mit 1 Substituenten Fluor substituiert sein können,
R³ für eine Gruppe der Formel 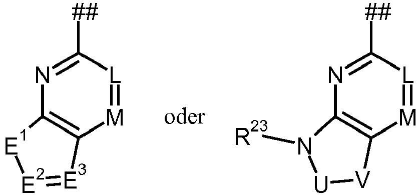 steht, wobei
## für die Anknüpfstelle an den Ring Q steht,
L für N steht
M für CR⁴ steht,
worin
R⁴ für -R⁵, -OR⁶ oder -NR⁷R⁸ steht,
worin
R⁵ für Trifluormethyl oder (C₁-C₄)-Alkyl steht,
worin (C₁-C₄)-Alkyl ihrerseits mit 1 oder 2 Substituenten unabhängig voneinander ausgewählt aus der Gruppe Trifluormethyl, Cyclopropyl, Cyclobutyl, Difluormethoxy, Trifluormethoxy, -(C=O)ₚ-OR⁹ und -C(=O)ₚ-NR⁹R¹⁰ substituiert sein kann,
worin
p die Zahl 0 bedeutet,
R⁹ und R¹⁰ unabhängig voneinander jeweils für Methyl, Ethyl, Trifluormethyl, 2,2,2-Trifluorethyl, 1,1,2,2,2-Pentafluorethyl, Cyclopropyl oder Cyclobutyl stehen, oder
R⁹ und R¹⁰ bilden zusammen mit dem/den Atom/-en, an die sie jeweils gebunden sind, einen Pyrrolidinyl-, Piperidinyl-, Piperazinyl- oder Morpholinyl-Ring, worin der Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁶ für (C₁-C₆)-Alkyl oder Pyrazolyl steht,
worin (C₁-C₆)-Alkyl mit 1 bis 3 Substituenten unabhängig voneinander ausgewählt aus der Gruppe Trifluormethyl, Cyclopropyl, Cyclobutyl, Cyclopentyl, Difluormethoxy, Trifluormethoxy, (C₁-C₄)-Alkoxy, -(C=O)-OR⁹ und -(C=O)-NR⁹R¹⁰ substituiert ist,
worin
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
und
worin Pyrazolyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl und Cyclopentyl substituiert sein können,
R⁷ für Wasserstoff, Methyl oder Ethyl steht,
R⁸ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Trifluormethyl, Cyclopropyl, Cyclobutyl, Trifluormethoxy, (C₁-C₄)-Alkoxy, Tetrahydrofuranyl, Pyrrolidinyl, Tetrahydropyranyl, Phenyl, Furanyl, Pyrazolyl, Imidazolyl, Triazolyl und Pyridyl substituiert ist,
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo, Difluormethoxy, Trifluormethoxy, Methoxy und Ethoxy substituiert sein können,
und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl,, Trifluormethoxy und Oxo substituiert sein können,
E¹ für NR¹⁸ steht,
worin
R¹⁸ für Wasserstoff steht,
- E²: für N steht,
- E³: für N oder CR¹⁹ steht,
worin
R¹⁹ für Wasserstoff, Fluor, Difluormethyl, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 0 oder 1 bedeutet,
R¹⁴ und R¹⁵ unabhängig voneinander jeweils für Wasserstoff, Methyl, Ethyl, Cyclopropyl, Cyclobutyl oder Phenyl stehen, worin Methyl und Ethyl weiterhin mit 1 oder 2 Substituenten unabhängig voneinander ausgewählt aus der Gruppe Fluor, Difluormethyl, Trifluormethyl, Cyclopropyl, Cyclobutyl, Hydroxy, Trifluormethoxy und Methoxy substituiert sein können, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmethyl und Cyclobutylmethyl substituiert sein können,
- U: für C=O steht,
- V: für NR²⁴ steht,
worin
R²⁴ für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-4-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Cyclopropyl und Cyclobutyl substituiert sein können,
R²³ für Wasserstoff steht,
sowie ihre Salze, Solvate und Solvate der Salze.

Weiterer Offenbarungsgegenstand sind Verbindungen der Formel (I), in welcher
- R³: für eine Gruppe der Formel 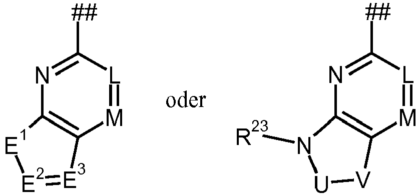 steht, wobei
## für die Anknüpfstelle an den Ring Q steht,
L für N steht
M für CR⁴ steht,
worin
R⁴ für -R⁵ oder -NR⁷R⁸ steht,
worin
R⁵ für Wasserstoff steht,
R⁷ für Wasserstoff steht,
R⁸ fürWasserstoff oder (C₁-C₆)-Alkyl steht,
worin (C₁-C₆)-Alkyl mit 1 bis 3 Substituenten unabhängig voneinander ausgewählt aus der Gruppe Fluor, Trifluormethyl, Cyclopropyl, Cyclobutyl, -(C=O)ₚ-OR⁹ und -(C=O)ₚ-NR⁹R¹⁰ substituiert ist,
worin
p die Zahl 0 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
E¹ für NR¹⁸ steht,
worin
R¹⁸ für Wasserstoff steht,
E² für N steht,
E³ für CR¹⁹ steht,
worin
R¹⁹ für Wasserstoff, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung steht,
R¹³ für -(C=O)ᵣ-OR¹⁴ oder -(C=O)ᵣ-NR¹⁴R¹⁵ steht,
worin
r die Zahl 0 bedeutet,
R¹⁴ und R¹⁵ unabhängig voneinander jeweils für Wasserstoff, Methyl, Ethyl, Cyclopropyl oder Cyclobutyl stehen,
U für C=O steht,
V für NR²⁴ steht,
worin
R²⁴ für (C₁-C₆)-Alkyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl und Hydroxy substituiert sein kann,
R²³ für Wasserstoff steht,
sowie ihre Salze, Solvate und Solvate der Salze.

Weiterer Offenbarungsgegenstand sind Verbindungen der Formel (I), in welcher
der Ring Q für eine Gruppe der Formel 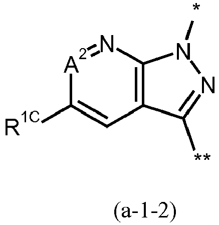 steht, wobei
- *: für die Anknüpfungsstelle an -CH₂-R² steht,
- **: für die Anknüpfungsstelle an den R³ steht,
- R^{1C}: für Wasserstoff oder Fluor steht,
- A²: für CH steht,
sowie ihre Salze, Solvate und Solvate der Salze.

Weiterer Offenbarungsgegenstand sind Verbindungen der Formel (I), in welcher
- R²: für 3,3,3-Trifluorprop-1-yl, 2,2,3,3-Tetrafluorprop-1-yl, 2,2,3,3,3-Pentafluorprop-1-yl, Phenyl, Pyridyl oder Pyrimidinyl steht,
wobei Phenyl mit 1 bis 3 Substituenten Fluor substituiert ist,
und
wobei Pyridyl und Pyrimidinyl mit 1 Substituenten Fluor substituiert sein können,
R³ für eine Gruppe der Formel 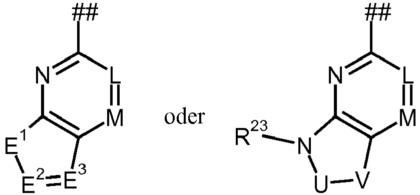 steht, wobei
## für die Anknüpfstelle an den Ring Q steht,
L für N steht
M für CR⁴ steht,
worin
R⁴ für -R⁵, -OR⁶ oder -NR⁷R⁸ steht,
worin
R⁵ für Trifluormethyl oder (C₁-C₄)-Alkyl steht,
worin (C₁-C₄)-Alkyl ihrerseits mit 1 oder 2 Substituenten unabhängig voneinander ausgewählt aus der Gruppe Trifluormethyl, Cyclopropyl, Cyclobutyl, Difluormethoxy, Trifluormethoxy, -(C=O)ₚ-OR⁹ und -C(=O)ₚ-NR⁹R¹⁰ substituiert sein kann,
worin
p die Zahl 0 bedeutet,
R⁹ und R¹⁰ unabhängig voneinander jeweils für Methyl, Ethyl, Trifluormethyl, 2,2,2-Trifluorethyl, 1,1,2,2,2-Pentafluorethyl, Cyclopropyl oder Cyclobutyl stehen, oder
R⁹ und R¹⁰ bilden zusammen mit dem/den Atom/-en, an die sie jeweils gebunden sind, einen Pyrrolidinyl-, Piperidinyl-, Piperazinyl- oder Morpholinyl-Ring,
worin der Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
R⁶ für (C₁-C₆)-Alkyl oder Pyrazolyl steht,
worin (C₁-C₆)-Alkyl mit 1 bis 3 Substituenten unabhängig voneinander ausgewählt aus der Gruppe Trifluormethyl, Cyclopropyl, Cyclobutyl, Cyclopentyl, Difluormethoxy, Trifluormethoxy, (C₁-C₄)-Alkoxy, -(C=O)-OR⁹ und -(C=O)-NR⁹R¹⁰ substituiert ist,
worin
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Pyrazolyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl und Cyclopentyl substituiert sein können,
R⁷ für Wasserstoff, Methyl oder Ethyl steht,
R⁸ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Trifluormethyl, Cyclopropyl, Cyclobutyl, Trifluormethoxy, (C₁-C₄)-Alkoxy, Tetrahydrofuranyl, Pyrrolidinyl, Tetrahydropyranyl, Phenyl, Furanyl, Pyrazolyl, Imidazolyl, Triazolyl und Pyridyl substituiert ist,
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo, Difluormethoxy, Trifluormethoxy, Methoxy und Ethoxy substituiert sein können,
und
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, , Trifluormethoxy und Oxo substituiert sein können,
E¹ für NR¹⁸ steht,
worin
R¹⁸ für Wasserstoff steht,
E² für N steht,
E³ für N oder CR¹⁹ steht,
worin
R¹⁹ für Wasserstoff, Fluor, Difluormethyl, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung, Methylen, Ethan-1,2-diyl oder Propan-1,3-diyl steht,
R¹³ für -(C=O)ᵣ-OR¹⁴, -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 0 oder 1 bedeutet,
R¹⁴ und R¹⁵ unabhängig voneinander jeweils für Wasserstoff, Methyl, Ethyl, Cyclopropyl, Cyclobutyl oder Phenyl stehen,
worin Methyl und Ethyl weiterhin mit 1 oder 2 Substituenten unabhängig voneinander ausgewählt aus der Gruppe Fluor, Difluormethyl, Trifluormethyl, Cyclopropyl, Cyclobutyl, Hydroxy, Trifluormethoxy und Methoxy substituiert sein können, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmethyl und Cyclobutylmethyl substituiert sein können,
U für C=O steht,
V für NR²⁴ steht,
worin
R²⁴ für Trideuteromethyl, (C₁-C₆)-Alkyl, Cyclopropyl, Cyclobutyl, Azetidin-3-yl, Pyrrolidin-3-yl oder Piperidin-4-yl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl, Hydroxy, Oxetanyl und Morpholin-4-yl substituiert sein kann,
und
worin Azetidin-3-yl, Pyrrolidin-3-yl und Piperidin-4-yl ihrerseits mit 1 oder 2 Substituenten unabhängig voneinander ausgewählt aus der Gruppe Fluor, Trifluormethyl, 2,2-Difluorethyl, 2,2,2-Trifluorethyl, Cyclopropyl und Cyclobutyl substituiert sein können,
R²³ für Wasserstoff steht,
sowie ihre Salze, Solvate und Solvate der Salze.

Weiterer Offenbarungsgegenstand sind Verbindungen der Formel (I), in welcher
- R²: für 3,3,3-Trifluorprop-1-yl, 2,2,3,3-Tetrafluorprop-1-yl, 2,2,3,3,3-Pentafluorprop-1-yl, 2-Fluorphenyl oder 3-Fluorpyrid-2-yl steht,
- R³: für eine Gruppe der Formel 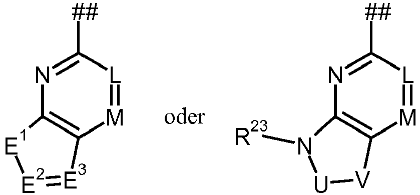 steht, wobei
## für die Anknüpfstelle an den Ring Q steht,
L für N steht
M für CR⁴ steht,
worin
R⁴ für -NR⁷R⁸ steht,
worin
R⁷ für Wasserstoff oder Methyl steht,
R⁸ für (C₁-C₆)-Alkyl steht,
worin (C₁-C₆)-Alkyl mit 1 bis 3 Substituenten unabhängig voneinander ausgewählt aus der Gruppe Trifluormethyl, Cyclopropyl, Cyclobutyl, Trifluormethoxy und (C₁-C₄)-Alkoxy substituiert sein ist,
E¹ für NR¹⁸ steht,
worin
R¹⁸ für Wasserstoff steht,
E² für N steht,
E³ für CR¹⁹ steht,
worin
R¹⁹ für Wasserstoff, Trifluormethyl, (C₁-C₄)-Alkyl oder eine Gruppe der Formel -M-R¹³ steht,
worin (C₁-C₄)-Alkyl mit 1 bis 3 Substituenten unabhängig voneinander ausgewählt aus der Gruppe Fluor, Trifluormethyl, Cyclopropyl und Cyclobutyl substituiert sein kann,
und worin
M für eine Bindung steht,
R¹³ für -(C=O)ᵣ-OR¹⁴ -(C=O)ᵣ-NR¹⁴R¹⁵ steht,
worin
r die Zahl 0 bedeutet,
R¹⁴ und R¹⁵ unabhängig voneinander jeweils für Wasserstoff, Methyl, Ethyl, Cyclopropyl oder Cyclobutyl stehen,
worin Methyl und Ethyl weiterhin mit 1 oder 2 Substituenten unabhängig voneinander ausgewählt aus der Gruppe Fluor, Difluormethyl, Trifluormethyl, Cyclopropyl, Cyclobutyl, Hydroxy, Trifluormethoxy und Methoxy substituiert sein können,
U für C=O steht,
V für NR²⁴ steht,
worin
R²⁴ für (C₁-C₆)-Alkyl steht,
worin (C₁-C₆)-Alkyl seinerseits mit 1 bis 3 Substituenten unabhängig voneinander ausgewählt aus der Gruppe Fluor, Trifluormethyl, Cyclopropyl, Cyclobutyl und Hydroxy substituiert sein kann,
R²³ für Wasserstoff steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Offenbarungsgegenstand ist ein Verfahren zur Herstellung der erfindungsgemäßen Verbindungen der Formel (I) dadurch gekennzeichnet, dass man
[A] eine Verbindung der Formel (II) 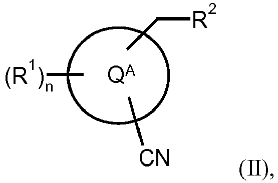 in welcher R² die oben angegebene Bedeutung hat und
   der Ring Q^{A} für eine Gruppe der Formel 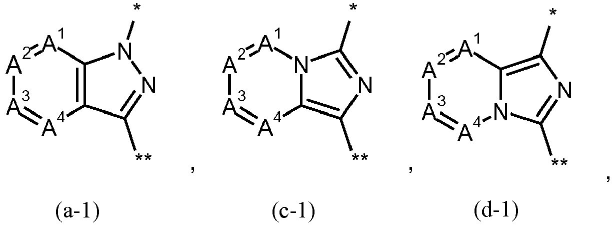 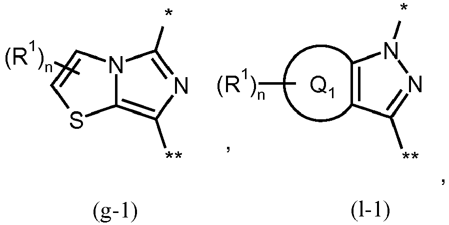 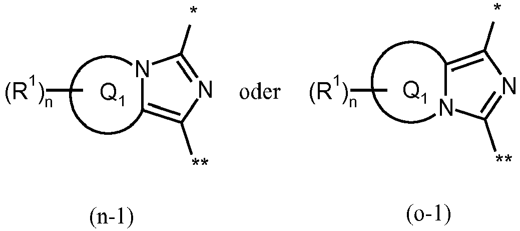 steht, wobei
   - *: für die Anknüpfungsstelle an -CH₂-R² steht,
   - **: für die Anknüpfungsstelle an den R³ steht,
   - der Ring Q₁: zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
   - R¹: für Fluor, Chlor, Methyl, Hydroxy oder Oxo steht,
   - n: für eine Zahl 0, 1 oder 2 steht,
   - A¹, A², A³ und A⁴: unabhängig voneinander jeweils für N, CH oder CR¹ stehen,
   mit der Maßgabe, dass maximal zwei der Gruppen A¹, A², A³ und A⁴ für N stehen,
   in einem inerten Lösungsmittel in Gegenwart einer geeigneten Base mit einer Verbindung der Formel (III-1) bzw. (III-2) 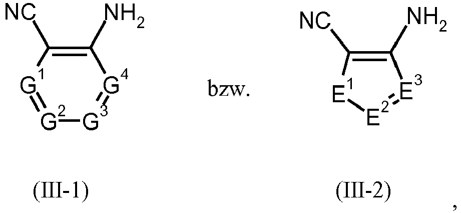 in welcher G¹, G², G³, G⁴, E¹, E² und E³ jeweils die oben angegebenen Bedeutungen haben,
   zu einer Verbindung der Formel (I-A-1) bzw- (I-A-2) 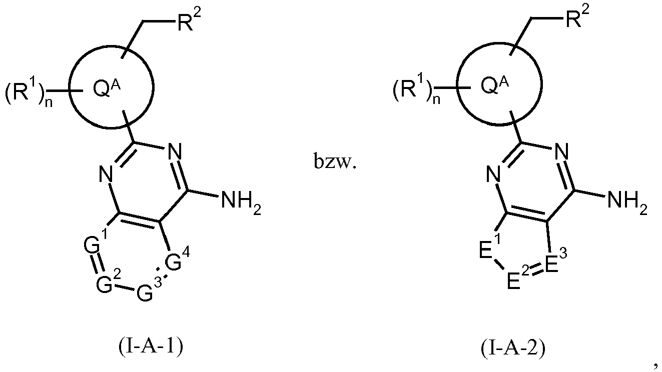 in welcher n, R¹, R², G¹, G², G³, G⁴, E¹, E², E³ und Q^{A} jeweils die oben angegebenen Bedeutungen haben,
   umsetzt, oder
[B] eine Verbindung der Formel (I-B-1) oder (I-B-2) 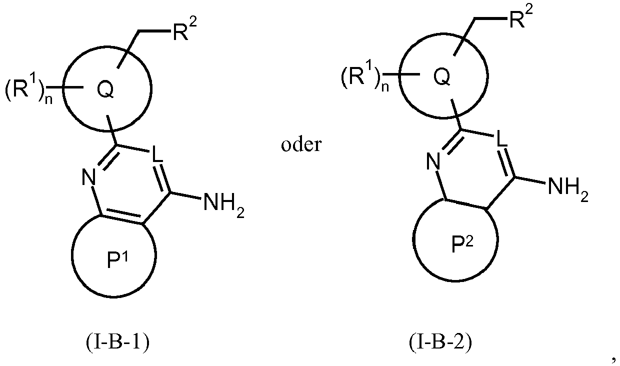 in welcher n, P¹, P², R¹, R² und Q jeweils die oben angegebenen Bedeutungen haben,
   in einem inerten Lösungsmittel mit einem geeigneten Nitrit zu einer Verbindung der Formel (I-I-3) oder (I-I-4) 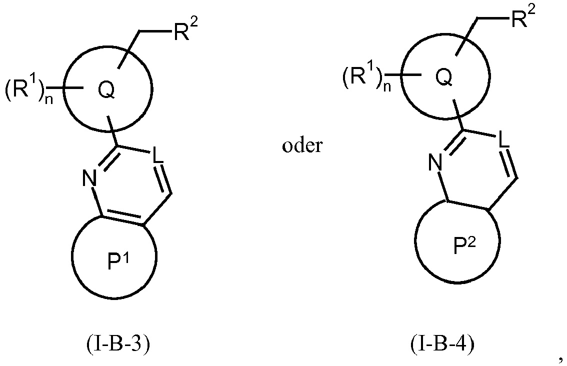 in welcher n, P¹, P², R¹, R² und Q jeweils die oben angegebenen Bedeutungen haben,
   umsetzt,
   oder
[C] eine Verbindung der Formel (I-B-1) oder (I-B-2) in einem inerten Lösungsmittel in eine Verbindung der Formel (IV-1) oder (IV-2) 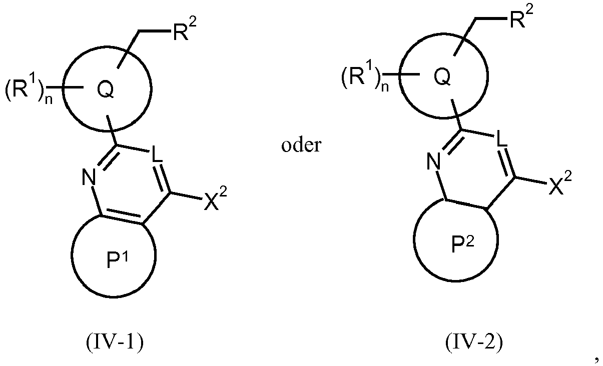 in welcher n, P¹, P², R¹, R² und Q jeweils die oben angegebenen Bedeutungen haben und
   - X²: für Brom, Iod oder Chlor steht,
   überführt, und diese im Anschluss in einen inerten Lösungsmittel gegebenenfalls in Gegenwart einer geeigneten Base mit einer Verbindung der Formel (V) 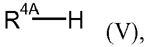 in welcher
   - R^{4A}: für -OR⁶ oder -NR⁷R⁸ steht,
   - wobei R⁶, R⁷ und R⁸: jeweils oben angegebenen Bedeutungen haben,
   zu einer Verbindung der Formel (IC-1) bzw. (I-C-2) 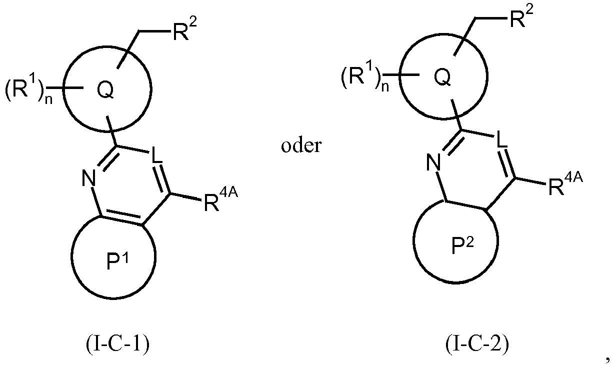 in welcher n, P¹, P², R¹, R², R^{4A} und Q jeweils die zuvor angegebenen Bedeutungen haben, oder
[D] eine Verbindung der Formel (VI) 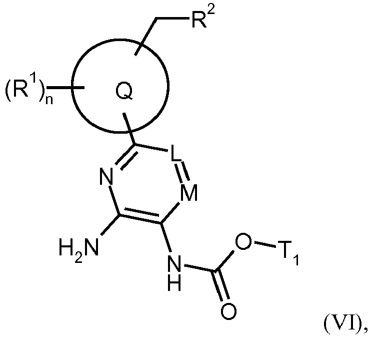 in welcher n, Q, R¹ und R² jeweils die oben angegebenen Bedeutungen haben und
   - T¹: für (C₁-C₄)-Alkyl steht,
   in einem inerten Lösungsmittel in Gegenwart einer geeigneten Base mit einer Verbindung der Formel (VII) 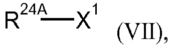 in welcher
   - R^{24A}: für Trideuteromethyl, (C₁-C₆)-Alkyl, (C₂-C₆)-Alkenyl, (C₃-C₇)-Cycloalkyl, Azetidinyl, Pyrrolidinyl, Piperidinyl oder Benzyl steht,
   worin (C₁-C₆)-Alkyl, Azetidinyl, Pyrrolidinyl, Piperidinyl und Benzyl im oben angegebenen Bedeutungsumfang substituiert sein können,
   und
   - X¹: für eine geeignete Abgangsgruppe, wie beispielsweise Halogen, insbesondere Chlor oder Brom, Meyslat oder Tosylat steht
zu einer Verbindung der Formel (VIII) 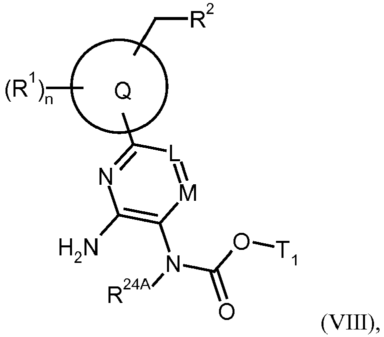 in welcher n, Q, R¹, R² und R^{24A} jeweils die oben angegebenen Bedeutungen haben,
umsetzt, und diese anschliessend in einem inerten Lösungsmittel in Gegenwart einer geeigneten Base zu einer Verbindung der Formel (I-D) 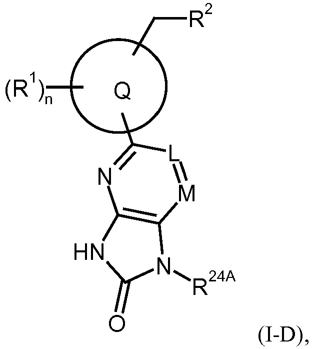 in welcher n, Q, R¹, R² und R^{24A} jeweils die oben angegebenen Bedeutungen haben,
cyclisiert,
und gegebenenfalls vorhandene Schutzgruppen nach den dem Fachmann bekannten Methoden abspaltet, und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Inerte Lösungsmittel für den Verfahrensschritt (II) + (III-1) → (I-A-1) bzw. (II) + (III-2) → (I-A-2) sind beispielsweise Halogenkohlenwasserstoffe wie Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid (DMF), Dimethylsulfoxid (DMSO), *N,N'*-Dimethylpropylenharnstoff (DMPU), *N-*Methylpyrrolidon (NMP), Sulfolan oder Pyridin. Ebenso ist es möglich, Gemische der genannten Lösungsmittel einzusetzen. Bevorzugt wird DMF verwendet.

Als Basen für den Verfahrensschritt (II) + (III-1) → (I-A-1) bzw. (II) + (III-2) → (I-A-2) eignen sich die üblichen anorganischen oder organischen Basen. Hierzu gehören bevorzugt Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid oder Amide wie Natriumamid, Lithium- oder Kalium-bis(trimethylsilyl)amid oder Lithiumdiisopropylamid, Bevorzugt wird Kalium-tert.-butylat verwendet.

Der Verfahrensschritt (II) + (111-1) → (I-A-1) bzw. (II) + (III-2) → (I-A-2) wird im Allgemeinen in einem Temperaturbereich von +100°C bis +200°C, bevorzugt bei +140°C bis +180°C durchgeführt, bevorzugt in einer Mikrowelle. Die Umsetzung kann bei normalem, erhöhtem oder bei erniedrigtem Druck erfolgen (z.B. von 0.5 bis 5 bar).

Geeignete Nitrite für die Umsetzungen (I-B-1) bzw. (I-B-2) → (I-B-3) bzw. (I-B-4) eind beispielsweise Natriumnitrit, Isopentylnitrit oder tert.-Butylnitrit.

Inerte Lösungsmittel für die Umsetzungen ((I-B-1) bzw. (I-B-2) → (I-B-3) bzw. (I-B-4) sind beispielsweise Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'*-Dimethylpropylenharnstoff (DMPU), *N-*Methylpyrrolidon (NMP), Pyridin oder Acetonitril. Ebenso ist es möglich, Gemische der genannten Lösungsmittel einzusetzen. Bevorzugt ist Tetrahydrofuran oder DMF.

Die Reaktionen (I-B-1) bzw. (I-B-2) → (I-B-3) bzw. (I-B-4) werden im Allgemeinen in einem Temperaturbereich von 0°C bis +120°C, bevorzugt bei +40°C bis +80°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (I-B-1) → (IV-1) bzw. (I-B-2) → (IV-2) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (I-B-1) → (IV-1) bzw. (I-B-2) → (IV-2) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (I-B-1) → (IV-1) bzw. (I-B-2) → (IV-2) erfolgt im Allgemeinen mit einem Molverhältnis von 10 bis 30 Mol Isopentylnitrit und 10 bis 30 Mol des Iod-Äquivalents bezogen auf 1 Mol der Verbindung der Formel (I-B-1) bzw. (I-B-2).

Als Halogen-Quelle bei der Umsetzung (I-B-1) → (IV-1) bzw. (I-B-2) → (IV-2) eignen sich beispielsweise Diiodmethan, eine Mischung aus Cäsiumiodid, Iod und Kupfer-(I)-iodid oder Kupfer -(II)-bromid sowie Phosphorylchlorid.

Inerte Lösungsmittel für den Verfahrensschritt (I-B-1) → (I-B-3) bzw. (I-B-2) → (I-B-4) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'*-Dimethylpropylenharnstoff (DMPU), *N-*Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion (I-B-1) → (I-B-3) bzw. (I-B-2) → (I-B-4) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (I-B-1) → (I-B-3) bzw. (I-B-2) → (I-B-4) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (IV-1) bzw. (IV-2) + (V) → (I-C-1) bzw. (I-C-2) sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N-*Methylpyrrolidon (NMP), Pyridin, Acetonitril oder Sulfolan. Ebenso ist es möglich, Gemische der genannten Lösungsmittel einzusetzen. Bevorzugt ist NMP.

Im Fall von R^{4A} = -OR⁶ erfolgt die Reaktion (IV-1) bzw. (IV-2) + (V) → (I-C-1) bzw. (I-C-2) bevorzugt ohne Lösungsmittel.

Im Fall von R^{4A} = -OR⁶ erfolgt die Umsetzung (IV-1) bzw. (IV-2) + (V) → (I-C-1) bzw. (I-C-2) in Gegenwart eines geeigneten Kupfer-Katalysators wie beispielsweise Kupfer-(I)-iodid, unter Zusatz von 3,4,7,8-Tetramethyl-1,10-Phenanthrolin, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Alternativ kann im Fall von R^{4A} = -OR⁶ die Darstellung der Verbindungen der Formel (I-C-1) bzw. (I-C-2) auch unter Mitsunobu-Bedingungen [siehe: a) Hughes, D. L. "The Mitsunobu Reaction," Organic Reactions; John Wiley & Sons, Ltd, 1992, vol. 42, p. 335. b) Hughes, D. L. Org. Prep. Proceed. Int. 1996, 28, 127.] ausgehend von einer Verbindung der Formel (IX-1) bzw. (IX-2) 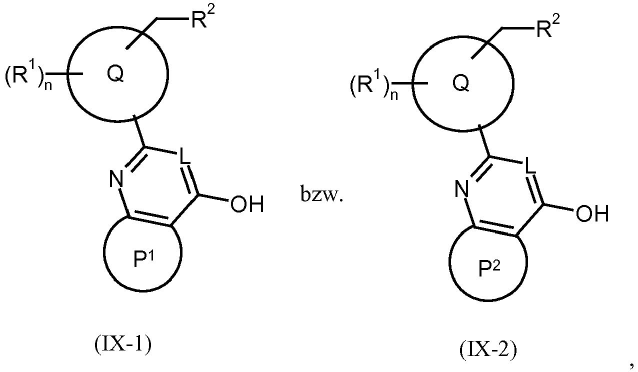 in welcher n, L, Q, P¹, P², R¹ und R² jeweils die oben angegebenen Bedeutungen haben,
erfolgen.

Hierbei erfolgt die Mitsunobu-Reaktion unter Verwendung von Triphenylphosphin, oder Tri-n-butylphosphin, 1,2-Bis(diphenylphosphino)ethan (DPPE), Diphenyl(2-pyridyl)phosphin (Ph2P-Py), (p-Dimethylaminophenyl)diphenylphosphin (DAP-DP), tris(4-Dimethylaminophenyl)-phosphin (tris-DAP) und eines geeigneten Dialkylazodicarboxylats, wie beispielsweise Diethylazodicarboxylat (DEAD), Diisopropylazodicarboxylat (DIAD), Di-tert-butylazodicarboxylat, N,N,N'N'-Tetramethylazodicarboxamid (TMAD), 1,1'-(Azodicarbonyl)-dipiperidin (ADDP) oder 4,7-Dimethyl-3,5,7-hexahydro-l,2,4,7-tetrazocin-3,8-dion (DHTD). Bervorzugt werden Triphenylphosphin und Diisopropylazodicarboxylat (DIAD) verwendet, oder eines geeigneten Azodicarbonamides, wie beispielsweise N,N,N',N'-Tetramethyldiazen-1,2-dicarboxamid.

Inerte Lösungsmittel für die Mitsunobu-Reaktion (IV-1) bzw. (IV-2) + (V) → (I-C-1) bzw. (I-C-2)sind beispielsweise Ether wie Tetrahydrofuran, Diethylether, Kohlenwasserstoffe wie Benzol, Toluol, Xylol, Halogenkohlenwasserstoffe wie Dichlormethan, Dichlorethan oder andere Lösungsmittel wie Acetonitril, DMF oder NMP. Ebenso ist es möglich, Gemische der genannten Lösungsmittel einzusetzen. Bevorzugt wird THF verwendet.

Die Mitsunobu-Reaktion (IV-1) bzw. (IV-2) + (V) → (I-C-1) bzw. (I-C-2)erfolgt im Allgemeinen in einem Temperaturbereich von -78°C bis +180°C, bevorzugt bei 0°C bis +50°C, gegebenenfalls in einer Mikrowelle. Die Umsetzungen können bei normalem, erhöhtem oder bei erniedrigtem Druck durchgeführt werden (z.B. von 0.5 bis 5 bar).

Im Fall von R^{4A} = -NR⁷R⁸, wenn R⁷ und R⁸ zusammen mit dem Stickstoffatom, an das sie gebunden sind, ein 5- oder 6-gliedriges Heteroaryl bilden, das im oben angegebenen Bedeutungsumfang substituiert sein kann, erfolgt die Umsetzung (IV-1) bzw. (IV-2) + (V) → (I-C-1) bzw. (I-C-2) in Gegenwart eines geeigneten Kupfer-Katalysators wie beispielsweise Kupfer-(I)-oxid, unter Zusatz von 2-Hydroxybenzaldehydoxim, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Die Reaktion (IV-1) bzw. (IV-2) + (V) → (I-C-1) bzw. (I-C-2) wird im Allgemeinen in einem Temperaturbereich von +20°C bis +200°C, bevorzugt bei +150°C bis +200°C, bevorzugt in einer Mikrowelle, durchgeführt. Die Umsetzung kann bei normalem, erhöhtem oder bei erniedrigtem Druck erfolgen (z.B. von 0.5 bis 5 bar).

Als inerte Lösungsmittel für die Cyclisierung (VIII) → (I-D) eignen sich Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), Dimethylacetamid, *N-*Methylpyrrolidon (NMP), Pyridin, Acetonitril, oder Sulfolan. Ebenso ist es möglich, Gemische der genannten Lösungsmittel einzusetzen. Bevorzugt ist Tetrahydrofuran.

Geeignete Basen für die Cyclisierung (VIII) → (I-D) sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N-*Methylmorpholin, *N-*Methylpiperidin, *N,N-*Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natrium-bis(trimethylsilyl)amid verwendet.

Die Reaktion (VIII) → (I-D) wird im Allgemeinen in einem Temperaturbereich von -10°C bis +80°C, bevorzugt bei +10°C bis +30°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (VI) + (VII) → (VIII) sind Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), Dimethylacetamid, *N-*Methylpyrrolidon (NMP), Pyridin, Acetonitril, oder Sulfolan. Ebenso ist es möglich, Gemische der genannten Lösungsmittel einzusetzen. Bevorzugt ist Tetrahydrofuran.

Geeignete Basen für die Umsetzung (VI) + (VII) → (VIII) sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N-*Methylmorpholin, *N-*Methylpiperidin, *N,N-*Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natriumhydrid verwendet.

Die Reaktion (VI) + (VII) → (VIII) wird im Allgemeinen in einem Temperaturbereich von -10°C bis +80°C, bevorzugt bei +10°C bis +30°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Geeignete Nitrite für die Umsetzungen (I-B-1) bzw. (I-B-2) → (I-B-3) bzw. (I-B-4) eind beispielsweise Natriumnitrit, Isopentylnitrit oder tert.-Butylnitrit.

Inerte Lösungsmittel für die Umsetzungen ((I-B-1) bzw. (I-B-2) → (I-B-3) bzw. (I-B-4) sind beispielsweise Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'*-Dimethylpropylenharnstoff (DMPU), *N-*Methylpyrrolidon (NMP), Pyridin oder Acetonitril. Ebenso ist es möglich, Gemische der genannten Lösungsmittel einzusetzen. Bevorzugt ist Tetrahydrofuran oder DMF.

Die Reaktionen (I-B-1) bzw. (I-B-2) → (I-B-3) bzw. (I-B-4) werden im Allgemeinen in einem Temperaturbereich von 0°C bis +120°C, bevorzugt bei +40°C bis +80°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (I-B-1) → (IV-1) bzw. (I-B-2) → (IV-2) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (I-B-1) → (IV-1) bzw. (I-B-2) → (IV-2) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (I-B-1) → (IV-1) bzw. (I-B-2) → (IV-2) erfolgt im Allgemeinen mit einem Molverhältnis von 10 bis 30 Mol Isopentylnitrit und 10 bis 30 Mol des Iod-Äquivalents bezogen auf 1 Mol der Verbindung der Formel (I-B-1) bzw. (I-B-2).

Als Iod-Quelle bei der Umsetzung (I-B-1) → (IV-1) bzw. (I-B-2) → (IV-2) eignen sich beispielsweise Diiodmethan oder eine Mischung aus Cäsiumiodid, Iod und Kupfer-(I)-iodid.

Inerte Lösungsmittel für den Verfahrensschritt (I-B-1) → (I-B-3) bzw. (I-B-2) → (I-B-4) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N'*-Dimethylpropylenharnstoff (DMPU), *N-*Methylpyrrolidon (NMP), Pyridin, Acetonitril oder auch Wasser. Ebenso ist es möglich, Gemische der genannten Lösungsmittel einzusetzen. Bevorzugt ist DMF.

Die Reduktion (I-B-1) → (I-B-3) bzw. (I-B-2) → (I-B-4)erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (I-B-1) → (I-B-3) bzw. (I-B-2) → (I-B-4) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Die zuvor beschriebenen Herstellverfahren werden anhand der nachfolgenden Syntheseschemata (Schema 1 und 2) beispielhaft erläutert: 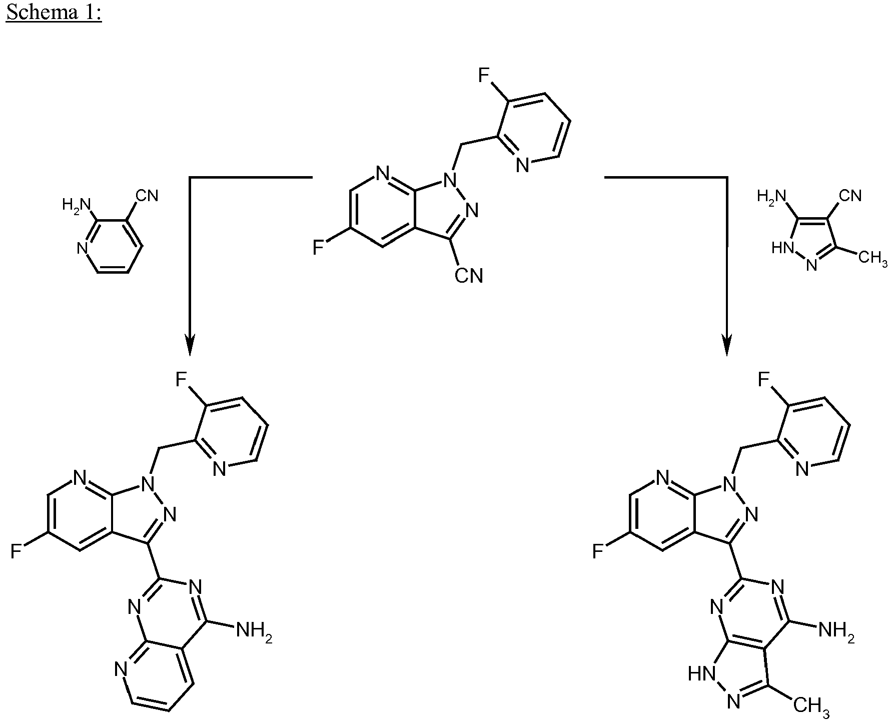 [Kalium-tert.-Butylat, DMF, Mikrowelle, 160°C]. 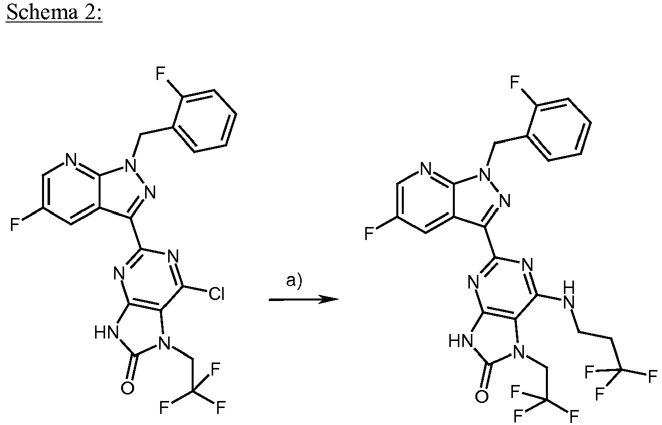 [a): 3,3,3-Trifluorpropyl-1-amin-Hydrochlorid, N,N-Diisopropylethylamin, NMP, Mikrowelle, 150°C.].

Die Verbindungen der Formeln (III-1), (III-2), (V) und (VII) sind kommerziell erhältlich, literaturbekannt oder können in Analogie zu literaturbekannten Verfahren hergestellt werden.

Die Verbindungen der Formeln (II), (I-B-1), (I-B-2), (IV-1), (IV-2) und (VI) sind literaturbekannt, können in Analogie zu literaturbekannten Verfahren oder wie im vorliegenden experimentellen Teil beschrieben hergestellt werden.

Die erfindungsgemäßen Verbindungen sind potente Stimulatoren der löslichen Guanylatcyclase, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens und/oder metabolischen Profils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Verwendung in der Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd/C: Palladium auf Aktivkohle (10%-ig)
- Ph: Phenyl
- RT: Raumtemperatur
- Rt: Retentionszeit (bei HPLC)
- t-Bu: tert.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-trüsopropylbiphenyl-2-yl)-phosphin

### HPLC- und LC/MS-Methoden:

### Methode 1 (LC-MS):

Instrument: Waters ACQUITY SQD UPLC System; Säule: Waters Acquity UPLC HSS T3 1,8µ 50 x 1mm; Eluent A: 11 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode2 (LC-MS):

Instrument: Waters ACQUITY SQD UPLC System; Säule: Waters Acquity UPLC HSS T3 1.8 µ 30 x 2 mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 3 (LC-MS):

Instrument: Micromass QuattroPremier mit Waters UPLC Acquity; Säule: Thermo Hypersil GOLD 1.9µ. 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Allgemeine Arbeitsvorschriften:

### Allgemeine Arbeitsvorschrift 1:

In einem Mikrowellengefäß mit Rührmagnet wurde 1.0 eq Beispiel 2A (100 mg, 0.369 mmol) zusammen mit 1.0 eq des entsprechenden Aminonitrils und 1.0 eq Kalium-tert.-butylat in Dimethylformamid (2.5 ml) gelöst, verschlossen und 2h bei 160° C unter Mikrowellenbestrahlung erhitzt. Danach erfolgte Reaktionskontrolle. Bei unvollständigem Umsatz wurden weitere 0.5 eq Kalium-tert.-butylat zugegeben und es wurde erneut bei 160° C unter Mikrowellenbestrahlung bis zum vollständigen Umsatz erhitzt. Die Reaktionsmischung wurde mittels präparativer HPLC gereinigt (Eluent: Acetonitril/Wasser mit 0.05% Ameisensäure, Gradient).

### Allgemeine Arbeitsvorschrift 2:

In einem Mikrowellengefäß mit Rührmagnet wurde 1.0 eq Beispiel 2A (100 mg, 0.32 mmol) zusammen mit 1.0 eq des entsprechenden Aminonitrils und 1.0 eq Kalium-tert.-butylat in Dimethylformamid (2 ml) gelöst, verschlossen und 2h bei 160° C unter Mikrowellenbestrahlung erhitzt. Danach erfolgte Reaktionskontrolle. Ggf (vgl. Beispiele) wurden weitere Reagenzien zugegeben und erneut unter Mikrowellenbestrahlung erhitzt. Die Reaktionsmischung wurde mittels präparativer HPLC gereinigt (Eluent: Acetonitril/Wasser mit 0.01% Ameisensäure, Gradient).

### Ausgangsverbindungen und Intermediate:

### Beispiel 1A

### 5-Fluor-1-[(3-fluorpyridin-2-yl)methyl]-3-iod-1H-pyrazolo[3,4-b]pyridin

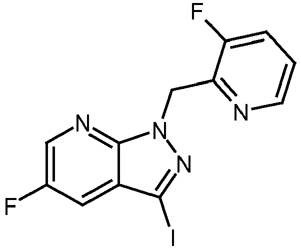

In DMF (10 ml) wurden 6.291 g (23.921 mmol) 5-Fluor-3-iod-1H-pyrazolo[3,4-b]pyridin (beschrieben in WO 2011/147809, Beispiel 1, Seite 42) und 8.573 g (26.313 mmol) Cäsiumcarbonat vorgelegt und anschließend 5.00 g (26.313 mmol) 2-(Brommethyl)-3-fluorpyridin in DMF (20 ml) gelöst zugetropft. Die Mischung wurde über Nacht bei RT gerührt. Anschließend wurde abgekühlt und auf 200 ml Wasser gegossen. Es wurde von einem Niederschlag abgesaugt, mit Wasser nachgewaschen und über Nacht im Hochvakuum getrocknet. Es wurden 6.28 g (70 % d. Th.) der Titelverbindung erhalten.
LC-MS (Methode 3): Rₜ = 2.17 min
MS (ESIpos): m/z = 373 (M+H)⁺
¹H-NMR (400 MHz, DMSO-d₆): δ = 5.88 (s, 2H), 7.42-7.46 (m, 1H), 7.77 (dd, 1H), 7.93 (dd, 1H), 8.27 (d, 1H), 8.67 (t, 1H).

### Beispiel 2A

### 5-Fluor-1-[(3-fluorpyridin-2-yl)methyl]-1H-pyrazolo[3,4-b]pyridin-3-carbonitril

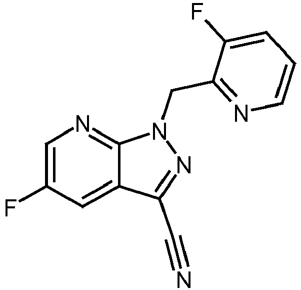

6.280 g (16.876 mmol) Beispiel 1A und 1.663 g (18.564 mmol) Kupfer-(I)-cyanid wurden in DMSO (100 ml) vorgelegt und 3 h bei 150°C gerührt. Nach Abkühlen wurde das Reaktionsgemisch über Celite filtriert und es wurde mit Essigsäureethylester nachgewaschen. Das Filtrat wurde viermal mit gesättigter wässriger Ammoniumchloridlösung und konz. Ammoniakwasser (3:1 v/v) extrahiert und die organische Phase abgetrennt. Diese wurde danach mit gesättigter wässriger Natriumchlorid-Lösung gewaschen, über Natriumsulfat getrocknet, filtriert und im Vakuum eingeengt. Es wurden 3.97 g (86 % d. Th.) der Titelverbindung erhalten.
LC-MS (Methode 1): Rₜ= 0.92 min
MS (ESIpos): m/z = 272 (M+H)⁺
¹H-NMR (400 MHz, DMSO-d₆): δ = 6.04 (s, 2H), 7.44-7.48 (m, 1H), 7.61 (t, 1H), 8.26 (d, 1H), 8.52 (dd, 1H), 8.83 (dd, 1H).

### Beispiel 3A

### Methyl-{4,6-diamino-2-[5-fluor-1-(2-fluorbenzyl)-1H-pyrazolo[3,4-b]pyridin-3-yl]pyrimidin-5-yl} (2,2,2-trifluorethyl)carbamat

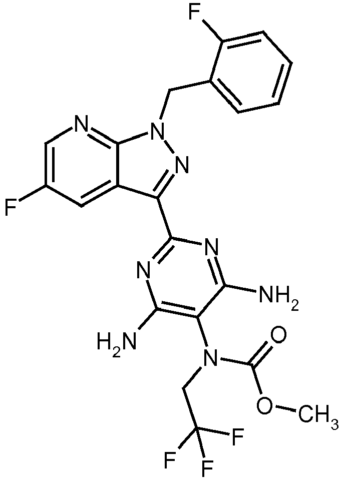

Die Synthese dieser Verbindung ist beschrieben in WO 2011/147809, Beispiel 3, Seite 44.

### Beispiel 4A

### 6-Amino-2-[5-fluor-1-(2-fluorbenzyl)-1H-pyrazolo[3,4-b]pyridin-3-yl]-7-(2,2,2-trifluorethyl)-7,9-dihydro-8H-purin-8-on

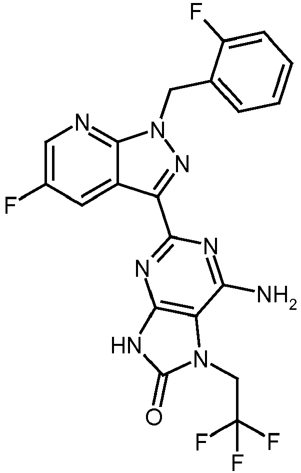

Unter einer Argon-Atmosphäre wurden 5.005 g (6.458 mmol) der Verbindung aus Beispiel 3A in 355 ml Tetrahydrofuran gelöst, auf 0°C gekühlt und mit 16.145 ml (16.145 mmol) einer IN Lösung von Bis-(trimethylsilyl)natriumamid in Tetrahydrofuran tropfenweise versetzt. Es wurde für 2 h bei 0°C und für 16 h bei RT gerührt. Es wurden 16.145 ml (16.145 mmol) IN Salzsäure addiert und das Gemisch am Rotationsverdampfer eingeengt. Der Rückstand wurde in Essigsäureethylester aufgenommen und die organische Phase zweimal mit Wasser gewaschen, über Natriumsulfat getrocknet und am Rotationsverdampfer eingeengt. Es wurden 6.13 g der Titelverbindung erhalten (Reinheit nach HPLC 61%). 500 mg des Rückstands wurden mittels präparativer HPLC gereinigt (Eluent: Methanol/Wasser, Gradient 30:70 → 90:10). Es wurden 93 mg der Titelverbindung erhalten (36% d. Th.).
LC-MS (Methode 1): Rₜ = 1.01 min; MS (EIpos): m/z = 477 [M+H]⁺.
¹H-NMR (400 MHz, DMSO-d₆): δ [ppm] = 4.91 (q, 2H), 5.80 (s, 2H), 7.01 (s br, 2H), 7.13-7.18 (m, 1H), 7.21-7.26 (m, 2H), 7.34-7.40 (m, 1H), 8.70 (dd, 1H), 8.87 (dd, 1H), 11.96 (s, 1H).

### Beispiel 5A

### 2-[5-Fluor-1-(2-fluorbenzyl)-1H-pyrazolo[3,4-b]pyridin-3-yl]-6-iod-7-(2,2,2-trifluorethyl)-7,9-dihydro-8H-purin-8-on

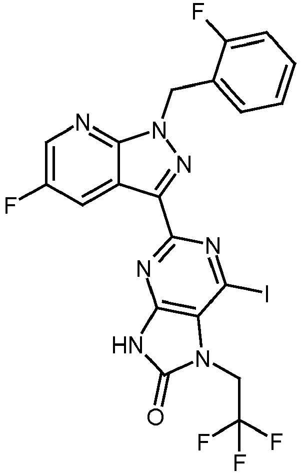

Es wurden 4.650 g (5.954 mmol) der Verbindung aus Beispiel 4A in 12 ml Diiodmethan gelöst und mit 12.76 ml (95.270 mmol) Isopentylnitrit versetzt. Es wurde für 16 h auf 85°C erhitzt und nach dem Abkühlen das Reaktionsgemisch am Rotationsverdampfer eingeengt. Es wurden 5 g des Rohprodukts (Reinheit 54%) erhalten. 1.2 g des Rückstands wurde mittels präparativer HPLC (Eluent: Acetonitril/Wasser mit 0.05% Ameisensäure, Gradient 40:60 → 95:5). Es wurden 128 mg der Titelverbindung erhalten (15% d. Th.).
LC-MS (Methode 1): Rₜ = 1.23 min; MS (EIpos): m/z = 588 [M+H]⁺.
¹H-NMR (400 MHz, DMSO-d₆): δ [ppm] = 4.95 (q, 2H), 5.86 (s, 2H), 7.14-7.18 (m, 1H), 7.21-7.27 (m, 2H), 7.35-7.41 (m, 1H), 8.47 (dd, 1H), 8.76 (dd, 1H), 12.79 (s, 1H).

### Beispiel 6A

### 6-Chlor-2-[5-fluor-1-(2-fluorbenzyl)-1H-pyrazolo[3,4-b]pyridin-3-yl]-7-(2,2,2-trifluorethyl)-7,9-dihydro-8H-purin-8-on

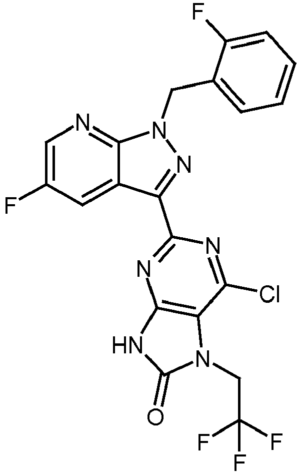

262 mg (0.446 mmol) der Verbindung aus Beispiel 5A wurden in 4 ml Phosphorylchlorid für 2 Stunden auf 85°C erhitzt. Anschliessend wurde der Ansatz auf warmes Wasser gegeben und für 1 Stunde nachgerührt. Es bildete sich ein Feststoff aus, welcher abfiltriert und mit Wasser nachgewaschen wurde. Man erhielt nach Trocknung im Hochvakuum 198 mg der Titelverbindung erhalten (89% d. Th.).
LC-MS (Methode 1): Rₜ = 1.29 min; MS (EIpos): m/z = 496 [M+H]⁺.
¹H-NMR (400 MHz, DMSO-d₆): δ [ppm] = 4.86 (q, 2H), 5.86 (s, 2H), 7.15-7.19 (m, 1H), 7.21-7.30 (m, 2H), 7.35-7.41 (m, 1H), 8.49 (dd, 1H), 8.77 (dd, 1H), 12.95 (s, 1H).

### Beispiel 7A

### 2-(2-Fluorphenyl)-N-[(6-oxo-1,4,5,6-tetrahydropyridazin-3-yl)methyl]acetamid

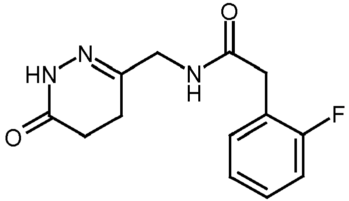

200.00 g (1.101 mol) Methyl-5-amino-4-oxopentanoat-Hydrochlorid wurden in Ethanol (3500 ml) vorgelegt, mit 64.28 ml (1.321 mol) Hydrazinhydrat versetzt und anschliessend 45 min zum Rückfluss erhitzt. Nach Abkühlen wurde Triethylamin (152 ml) zugegeben und das Gemisch bis zur Trockene eingeengt. Zum Rückstand wurde Wasser (500 ml) gegeben und eingeengt. Danach wurde Ethanol (500 ml) zugegeben, eingeengt, danach zweimal Toluol (500 ml) zugegeben und bis zur Trockene eingeengt. Der Rückstand (140 g) wurde in Acetonitril (500 ml) gelöst und langsam bei 0°C zu einer Lösung von 307.85 g (1.784 mol) (2-Fluorphenyl)acetylchlorid (Herstellung: Journal of Organic Chemistry; 22; 1957; 879) und 304.86 ml (2.202 mol) Triethylamin in Acetonitril (1500 ml) und Molsieb getropft. Es wurde 3 Tage bei 20°C gerührt. Danach wurde filtriert und der Niederschlag wurde mit tert.-Butylmethylether gewaschen und anschliessend getrocknet. Es wurden 458 g der Zielverbindung erhalten (90 % d. Th.).
LC-MS (Methode 1): Rₜ = 0.57 min; MS (EIpos): m/z = 264 [M+H]⁺.

### Beispiel 8A

### 2-(2-Fluorphenyl)-N-[(6-oxo-1,6-dihydropyridazin-3-yl)methyl]acetamid

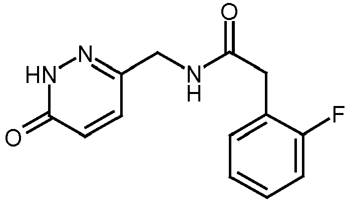

458 g (1.740 mol) der unter Beispiel 7A erhaltenen Verbindung wurden in Essigsäure (2250 ml) vorgelegt und auf 50°C erwärmt. Bei dieser Temperatur wurde unter starkem Rühren 98.16 ml (1.914 mol) Brom zugetropft und anschliessend 3h bei 50°C weitergerührt. Nach Abkühlen wurde das Reaktionsgemisch bis zur Trockene eingeengt. Der Rückstand wurde mit gesättigter wässriger Natriumhydrogencarbonatlösung (4800 ml) verrührt. Danach wurde filtriert und der Niederschlag wurde mit wenig Wasser gewaschen. Das Filtrat wurde zweimal mit Essigsäureethylester extrahiert. Die organischen Phasen wurden vereinigt, getrocknet und eingeengt. Es wurden 117 g der Zielverbindung erhalten (25 % d. Th.).
LC-MS (Methode 1): Rₜ = 0.56 min; MS (EIpos): m/z = 262 [M+H]⁺.
¹H-NMR (400 MHz, DMSO-d₆): δ [ppm] = 3.54 (s, 2H), 4.16 (d, 2H), 6.86 (d, 1H), 7.12-7.16 (m, 2H), 7.27-7.35 (m, 3H), 8.62 (t, 1H), 12.88 (s, 1H).

### Beispiel 9A

### 2-Chlor-7-(2-fluorbenzyl)imidazo[1,5-b]pyridazin

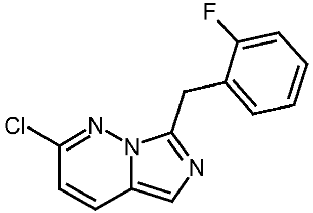

65.00 g (248.79 mmol) der unter Beispiel 8A erhaltenen Verbindung wurden in Sulfolan (780 ml) vorgelegt, mit 185.52 ml (1.990 mol) Phosphoroxychlorid versetzt und anschliessend 3h bei 100°C gerührt. Danach wurde am Hochvakuum der Überschuss an Phosphoroxychlorid abdestilliert, der Rückstand in Essigsäureethylester aufgenommen und zu einer gesättigten wässrigen Natriumhydrogencarbonatlösung gegeben. Es wurde mit Wasser verdünnt und danach mit Essigsäureethylester extrahiert. Die organischen Phasen wurden vereinigt, mit Wasser gewaschen, über Natriumsulfat getrocknet und eingeengt. Der Rückstand wurde mittels Chromatographie an Kieselgel (Laufmittel: Dichlormethan/Methanol 20:1 → 5:1 (v/v)) gereinigt, anschliessend mit Wasser gewaschen und mittels Chromatographie an Kieselgel (Laufmittel: Dichlormethan/Methanol 100:1 v/v) gereinigt. Es wurden 23.6 g der Zielverbindung erhalten (36 % d. Th.).
LC-MS (Methode 1): Rₜ = 1.00 min; MS (EIpos): m/z = 262 [M+H]⁺.
¹H-NMR (400 MHz, DMSO-d₆): δ [ppm] = 4.40 (s, 2H), 6.84 (d, 1H), 7.10-7.33 (m, 4H), 7.55 (s, 1H), 8.19 (d, 1H).

### Beispiel 10A

### 7-(2-Fluorbenzyl)imidazo[1,5-b]pyridazin

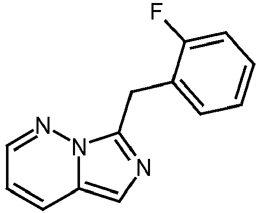

2.004 g Palladium auf Kohle (5%-ig) wurden unter Argon vorgelegt und anschliessend mit 20.04 g (76.58 mmol) der unter Beispiel 9A erhaltenen Verbindung in Essigsäureethylester (750 ml) versetzt. Danach wurde 21.348 ml (153.159 mmol) Triethylamin zugegeben und das Reaktionsgemisch wurde 16 Stunden bei Wasserstoff-Normaldruck und 20°C hydriert. Anschliessend wurde das Reaktionsgemisch abermals mit der oben angegebenen Menge an Katalysator versetzt und eine weitere Nacht bei Wasserstoff-Normaldruck und 20°C hydriert. Im Anschluss daran wurde über Celite filtriert, mit Ethanol nachgewaschen, eingeengt und am Hochvakuum getrocknet. Es wurden 22.79 g der Zielverbindung erhalten (ca. 100 % d. Th., verunreinigt mit Triethylamin).
LC-MS (Methode 1): Rₜ = 0.77 min; MS (EIpos): m/z = 228 [M+H]⁺.
¹H-NMR (400 MHz, DMSO-d₆): δ [ppm] = 4.44 (s, 2H), 6.70 (dd, 1H), 7.08-7.31 (m, 4H), 7.45 (s, 1H), 8.09 (dd, 1H), 8.28 (dd, 1H).

### Beispiel 11A

### 5-Brom-7-(2-fluorbenzyl)imidazo[1,5-b]pyridazin

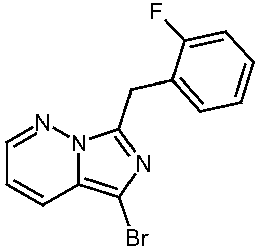

22.46 g (98.837 mmol) der unter Beispiel 10A erhaltenen Verbindung wurden in Dichlormethan (400 ml) vorgelegt und mit 17.591 g (98.837 mmol) N-Bromsuccinimid versetzt. Danach wurde das Gemisch 10 min bei 20°C gerührt. Anschliessend wurde das Reaktionsgemisch mit Wasser versetzt, die Phasen wurden separiert und die organische Phase wurde mit Wasser gewaschen. Die wässrige Phase wurde zweimal mit Dichlormethan extrahiert, die vereinigten organischen Phasen wurden mit gesättigter wässriger Natriumchloridlösung gewaschen, über Natriumsulfat getrocknet, filtriert und eingeengt. Es wurden 22.78 g der Zielverbindung erhalten (75% d. Th.).
LC-MS (Methode 1): Rₜ = 1.05 min; MS (EIpos): m/z = 306, 308 [M+H, Brom-Muster]⁺.
¹H-NMR (400 MHz, DMSO-d₆): δ [ppm] = 4.45 (s, 2H), 6.81 (dd, 1H), 7.12-7.34 (m, 4H), 7.94 (dd, 1H), 8.28 (dd, 1H).

### Beispiel 12A

### 7-(2-Fluorbenzyl)imidazo[1,5-b]pyridazin-5-carbonitril

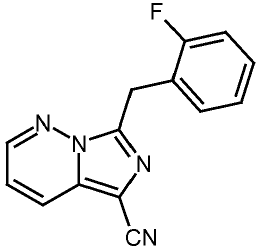

1.00 g (3.266 mmol) der unter Beispiel 11A erhaltenen Verbindung wurden in trockenem DMSO (25 ml) vorgelegt, mit 1.170 g (13.066 mmol) Kupfer-(I)-cyanid versetzt und 3.5h unter Rühren auf 170°C erhitzt. Danach wurde über Celite filtriert und mit Essigsäureethylester und Tetrahydrofuran nachgewaschen. Anschliessend wurde das Filtrat viermal mit einer Mischung aus gesättiger wässriger Ammoniumchlorid-Lösung/Ammoniakwasser (33 %) (3:1, v/v) extrahiert und einmal mit gesättigter wässriger Natriumchlorid-Lösung gewaschen. Die Phasen wurden separiert und die organische Phase wurde mit Natriumsulfat getrocknet, filtriert und eingeengt. Der Rückstand wurde im Ultraschallbad mit Ethanol aufgeschlossen und danach mit Wasser versetzt. Der sich bildende Niederschlag wurde abfiltriert, mit Ethanol nachgewaschen und anschliessend am Hochvakuum getrocknet. Es wurden 586 mg der Zielverbindung erhalten (71% d. Th.).
LC-MS (Methode 1): Rₜ = 0.95 min; MS (EIpos): m/z = 253 [M+H]⁺.
¹H-NMR (400 MHz, DMSO-d₆): δ [ppm] = 4.49 (s, 2H), 7.13-7.35 (m, 5H), 8.40 (d, 1H), 8.61 (d, 1H).

### Beispiel 13A

### 5-Fluor-3-iod-1-(3,3,4,4,4-pentafluorbutyl)-1H-pyrazolo[3,4-b]pyridin

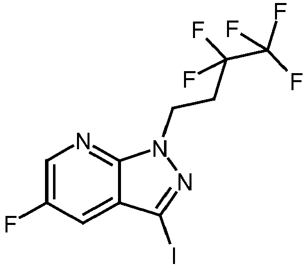

In DMF (100 ml) wurden 5.0 g (19.010 mmol) 5-Fluor-3-iod-1H-pyrazolo[3,4-b]pyridin vorgelegt und anschließend 20.83 g (76.042 mmol) 1,1,1,2,2-Pentafluor-4-iodbutan sowie 14.86 g (45.65 mmol) Cäsiumcarbonat und 0.63 g (3.802 mmol) Kaliumiodid zugefügt. Die Mischung wurde über Nacht bei 140°C gerührt. Anschließend wurde abgekühlt und mit einem Vorversuch vereinigt, der analog ausgehend von 200 mg 5-Fluor-3-iod-1H-pyrazolo[3,4-b]pyridin durchgeführt wurde. Es wurde von Feststoffen abgesaugt, mit DMF nachgewaschen, und das Filtrat anschliessend im Hochvakuum eingeengt. Der Rückstand wurde mittels präparativer HPLC gereinigt (Methanol:Wasser - Gradient). Man erhielt 4.34 g (52 % d. Th.) der Titelverbindung.
LC-MS (Methode 2): Rₜ = 1.30 min
MS (ESIpos): m/z = 410 (M+H)⁺
¹H-NMR (400 MHz, DMSO-d₆): δ = 2.84-3.00 (m, 2H), 4.79 (t, 2H), 7.93 (dd, 1H), 8.71 (dd, 1H).

### Beispiel 14A

### 5-Fluor-1-(3,3,4,4,4-pentafluorbutyl)-1H-pyrazolo[3,4-b]pyridin-3-carbonitril

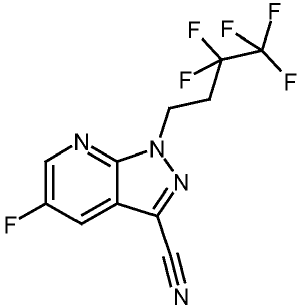

Eine Suspension aus 4.34 g (10.609 mmol) Beispiel 13A und 1.045 g (11.670 mmol) Kupfer-(I)-cyanid wurden in DMSO (30 ml) vorgelegt und 2 h bei 150°C gerührt. Nach Abkühlen wurde der Ansatz über Celite filtriert, mit Essigsäureethylester und THF nachgewaschen und anschliessend viermal mit einer Lösung aus gesättigter wässriger Ammoniumchloridlösung und konz. Ammoniakwasser (3:1 v/v) extrahiert. Die vereinigten organischen Phasen wurden mit ges. wässriger Natriumchlorid-Lösung gewaschen, über Natriumsulfat getrocknet, filtriert und im Vakuum eingeengt.
Ausbeute: 3.19 g (97 % d. Theorie)
¹H-NMR (400 MHz, DMSO-d₆): δ = 2.94-3.09 (m, 2H), 4.93 (t, 2H), 8.54 (dd, 1H), 8.88 (dd, 1H).

### Ausführungsbeispiele:

### Beispiel 1

### 6-{5-Fluor-1-[(3-fluorpyridin-2-yl)methyl]-1H-pyrazolo[3,4-b]pyridin-3-yl}-3-methyl-1H-pyrazolo[3,4-d]pyrimidin-4-amin

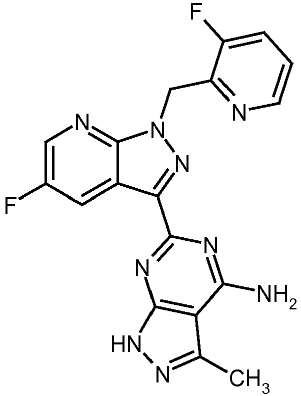

Nach der Allgemeinen Arbeitsvorschrift 1 wurden 100 mg (0.369 mmol) Beispiel 2A mit 5-Amino-3-methyl-1H-pyrazol-4-carbonitril umgesetzt.
Ausbeute: 14 mg (10 % d. Th.)
LC-MS (Methode 1): Rₜ = 0.71 min; MS (EIpos): m/z = 394 [M+H]⁺.
¹H-NMR (400 MHz, DMSO-d₆): δ [ppm] = Methylgruppe, 3H wahrscheinlich unter DMSO-Signal, 5.96 (s, 2H), 7.42-7.46(m, 1H), 7.76 (t, 1H), 8.28 (d, 1H), 8.67 (s br, 1H), 8.90 (dd, 1H), 12.97 (s br, 1H).

### Beispiel 2

### N³,N³-Diethyl-6-{5-fluor-1-[(3-fluorpyridin-2-yl)methyl]-1H-pyrazolo[3,4-b]pyridin-3-yl}-1H-pyrazolo[3,4-d]pyrimidin-3,4-diamin

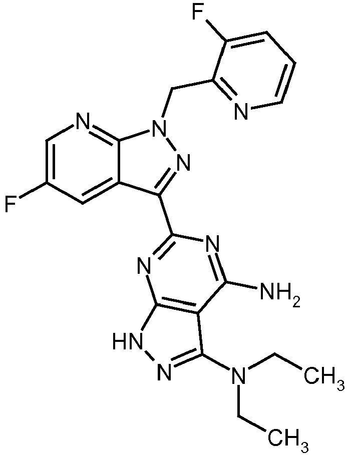

Nach der Allgemeinen Arbeitsvorschrift 1 wurden 100 mg (0.369 mmol) Beispiel 2A mit 5-Amino-3-(diethylamino)-1H-pyrazol-4-carbonitril umgesetzt.
Ausbeute: 37 mg (22 % d. Th.)
LC-MS (Methode 1): Rₜ = 0.93 min; MS (EIpos): m/z = 451 [M+H]⁺.
¹H-NMR (400 MHz, DMSO-d₆): δ [ppm] = 1.04 (t, 6H), 3.22 (q, 4H), 5.96 (s, 2H), 7.42-7.46(m, 1H), 7.75-7.80 (m, 1H), 8.27-8.29 (m, 1H), 8.66 (dd, 1H), 8.90 (dd, 1H), 12.55 (s br, 1H).

### Beispiel 3

### 2-{5-Fluor-1-[(3-fluorpyridin-2-yl)methyl]-1H-pyrazolo[3,4-b]pyridin-3-yl}pyrido[2,3-d]pyrimidin-4-amin

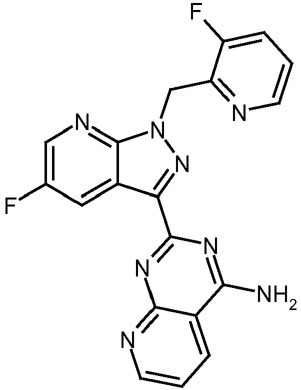

Nach der Allgemeinen Arbeitsvorschrift 1 wurden 100 mg (0.369 mmol) Beispiel 2A mit 2-Amino-pyridincarbonitril umgesetzt.
Ausbeute: 19 mg (13 % d. Th.)
LC-MS (Methode 1): Rₜ = 0.71 min; MS (EIpos): m/z = 391 [M+H]⁺.
¹H-NMR (400 MHz, DMSO-d₆): δ [ppm] = 6.01 (s, 2H), 7.43-7.47 (m, 1H), 7.52 (dd, 1H), (7.76-7.81 (m, 1H), 8.28-8.30 (m, 1H), 8.33 (s br, 2H), 8.67-8.70 (m, 2H), 8.90 (dd, 1H), 9.01 (dd, 1H).

### Beispiel 4

### 6-{5-Fluor-1-[(3-fluorpyridin-2-yl)methyl]-1H-pyrazolo[3,4-b]pyridin-3-yl}-3-methoxy-1H-pyrazolo[3,4-d]pyrimidin-4-amin

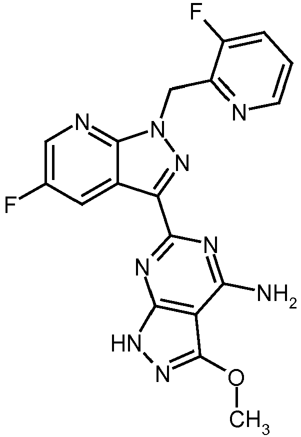

Nach der Allgemeinen Arbeitsvorschrift 1 wurden 100 mg (0.369 mmol) Beispiel 2A mit 5-Amino-3-methoxy-1H-pyrazol-4-carbonitril umgesetzt.
Ausbeute: 18 mg (12 % d. Th.)
LC-MS (Methode 1): Rₜ = 0.79 min; MS (EIpos): m/z = 410 [M+H]⁺.
¹H-NMR (400 MHz, DMSO-d₆): δ [ppm] = 3.98 (s, 3H), 5.96 (s, 2H), 7.42-7.46 (m, 1H), 7.75-7.80 (m, 1H), 8.27-8.29 (m, 1H), 8.66 (dd, 1H), 8.88 (dd, 1H), 12.41 (s br, 1H).

### Beispiel 5

### 6-[(Cyclopropylmethyl)amino]-2-[5-fluor-1-(2-fluorbenzyl)-1H-pyrazolo[3,4-b]pyridin-3-yl]-7-(2,2,2-trifluorethyl)-7,9-dihydro-8H-purin-8-on

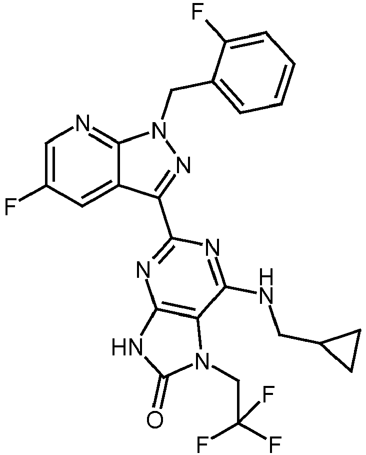

In einem Mikrowellengefäß mit Rührmagnet wurden 90 mg, (0.182 mmol) Beispiel 6A in 2 ml NMP gelöst und mit Aminomethylcyclopropan (0.5 ml) versetzt und anschliessend 3h bei 150° C unter Mikrowellenbestrahlung erhitzt. Die Reaktionsmischung wurde mittels präparativer HPLC gereinigt (Eluent: Acetonitril/Wasser mit 0.05% Ameisensäure, Gradient).
Ausbeute: 18 mg (18 % d. Th.)
LC-MS (Methode 1): Rₜ = 1.26 min; MS (EIpos): m/z = 531 [M+H]⁺.
¹H-NMR (400 MHz, DMSO-d₆): δ [ppm] = 0.32-0.35 (m, 2H), 0.43-0.48 (m, 2H), 1.23-1.28 (m, 1H), 3.51 (dd, 2H), 4.96 (q, 2H), 5.82 (s, 2H), 6.91 (t, 1H), 7.13-7.17 (m, 1H), 7.20-7.27 (m, 2H), 7.35-7.40 (m, 1H), 8.57 (dd, 1H), 8.72 (dd, 1H), 11.99 (s, 1H).

### Beispiel 6

### 2-[5-Fluor-1-(2-fluorbenzyl)-1H-pyrazolo[3,4-b]pyridin-3-yl]-7-(2,2,2-trifluorethyl)-6-[(3,3,3-trifluorpropyl)amino]-7,9-dihydro-8H-purin-8-on

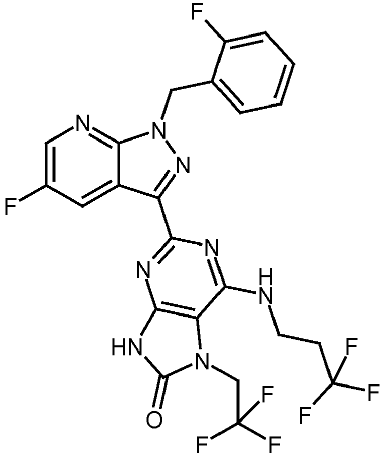

In einem Mikrowellengefäß mit Rührmagnet wurden 90 mg (0.182 mmol) Beispiel 6A in 2 ml NMP gelöst und mit 122 mg (0.817 mmol) 3,3,3-Trifluorpropyl-1-amin-Hydrochlorid und mit 0.164 ml (0.944 mmol) N,N-Diisopropylethylamin versetzt und anschliessend 3h bei 150° C unter Mikrowellenbestrahlung erhitzt. Die Reaktionsmischung wurde mittels präparativer HPLC gereinigt (Eluent: Acetonitril/Wasser mit 0.05% Ameisensäure, Gradient). Es wurden 54 mg isoliert, die noch Startmaterial enthielten. Daraufhin wurden 44 mg dieses Gemischs in einem Mikrowellengefäß mit Rührmagnet in 1 ml NMP gelöst und mit 60 mg (0.405 mmol) 3,3,3-Trifluorpropyl-1-amin-Hydrochlorid und mit 81 µl (0.468 mmol) N,N-Diisopropylethylamin versetzt und anschliessend 6h bei 150° C unter Mikrowellenbestrahlung erhitzt. Die Reaktionsmischung wurde mittels präparativer HPLC gereinigt (Eluent: Acetonitril/Wasser mit 0.05% Ameisensäure, Gradient).
Ausbeute: 34 mg (33 % d. Th.)
LC-MS (Methode 1): Rₜ = 1.22 min; MS (EIpos): m/z = 573 [M+H]⁺.
¹H-NMR (400 MHz, DMSO-d₆): δ [ppm] = 2.65-2.72 (m, 2H), 3.85-3.90 (q, 2H), 4.90-4.97 (q, 2H), 5.82 (s, 2H), 6.97 (t, 1H), 7.16 (t, 1H), 7.20-7.29 (m, 2H), 7.34-7.40 (m, 1H), 8.46 (dd, 1H), 8.72 (dd, 1H), 12.09 (s, 1H).

### Beispiel 7

### 6-[7-(2-Fluorbenzyl)imidazo[1,5-b]pyridazin-5-yl]-3-methyl-1H-pyrazolo[3,4-d]pyrimidin-4-amin

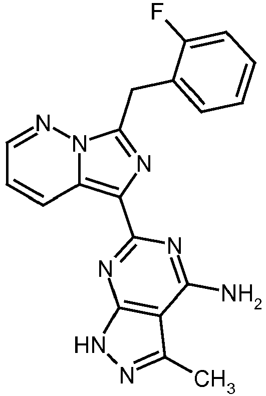

Nach der Allgemeinen Arbeitsvorschrift 1 wurden 100 mg (0.369 mmol) Beispiel 12A mit 5-Amino-3-methyl-1H-pyrazol-4-carbonitril bei 200°C umgesetzt.
Ausbeute: 19 mg (13 % d. Th.)
LC-MS (Methode 1): Rₜ = 0.77 min; MS (EIpos): m/z = 375 [M+H]⁺.
¹H-NMR (400 MHz, DMSO-d₆): δ [ppm] = Methylgruppe, 3H wahrscheinlich unter DMSO-Signal, 4.50 (s, 2H), 6.95 (dd, 1H), 7.12-7.23 (m, 2H), 7.29-7.34 (m, 2H), 8.41 (dd, 1H), 9.11 (dd, 1H), 12.80 (s br, 1H).

### Beispiel 8

### 6-[5-Fluor-1-(3,3,4,4,4-pentafluorbutyl)-1H-pyrazolo[3,4-b]pyridin-3-yl]-3-methyl-1H-pyrazolo[3,4-d]pyrimidin-4-amin

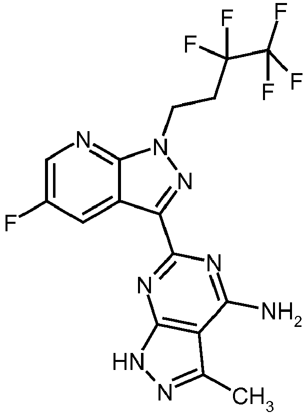

Nach der Allgemeinen Arbeitsvorschrift 2 wurden 200 mg (0.65 mmol) Beispiel 14A, 5-Fluor-1-(3,3,4,4,4-pentafluorbutyl)-1H-pyrazolo[3,4-b]pyridin-3-carbonitril, mit 5-Amino-3-methyl-1H-pyrazol-4-carbonitril umgesetzt.
Ausbeute: 6.6 mg (2 % d. Th.)
LC-MS (Methode 1): Rₜ = 0.92 min; MS (EIpos): m/z = 431 [M+H]⁺.
¹H-NMR (400MHz, DMSO-d₆): δ [ppm]= 2.57 (s, 3H), 2.90 - 3.12 (m, 2H), 4.88 (t, 2H), 7.50 (br. s, 2H), 8.68 - 8.74 (m, 1H), 8.94 (dd, 1H), 13.03 (br. s, 1H).

### Beispiel 9

### N3,N3-Diethyl-6-{5-fluor-1-[(3-fluorpyridin-2-yl)methyl]-1H-pyrazolo[3,4-b]pyridin-3-yl}-1H-pyrazolo[3,4-d]pyrimidin-3,4-diamin

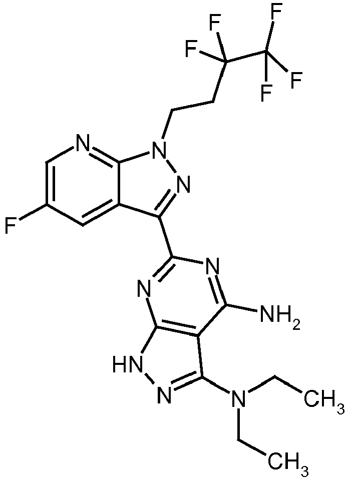

Nach der Allgemeinen Arbeitsvorschrift 2 wurden 100 mg (0.32 mmol) Beispiel 14A, 5-Fluor-1-(3,3,4,4,4-pentafluorbutyl)-1H-pyrazolo[3,4-b]pyridin-3-carbonitril, mit 5-Amino-3-(diethyl-amino)-1H-pyrazol-4-carbonitril umgesetzt. Danach wurden weitere 2 h bei 200° C und schließlich 10 h bei 160° C unter Mikrowellenbestrahlung erhitzt.
Ausbeute: 5.2 mg (3 % d. Th.)
LC-MS (Methode 1): Rₜ = 1.09 min; MS (EIpos): m/z = 488 [M+H]⁺.
¹H-NMR (400MHz, DMSO-d₆): δ [ppm]= 1.05 (t, 6H), 2.86 - 3.13 (m, 3H), 3.22 (q, 4H), 4.88 (t, 2H), 7.22 (br. s, 2H), 8.70 (dd, 1H), 8.95 (dd, 1H), 12.61 (s, 1H).

### Beispiel 10

### 2-[5-Fluor-1-(3,3,4,4,4-pentafluorbutyl)-1H-pyrazolo[3,4-b]pyridin-3-yl]pyrido[2,3-d]pyrimidin-4-amin

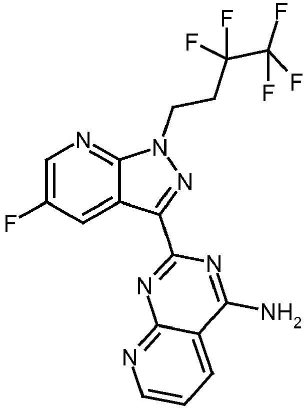

Nach der Allgemeinen Arbeitsvorschrift 2 wurden 200 mg (0.65 mmol) Beispiel 14A, 5-Fluor-1-(3,3,4,4,4-pentafluorbutyl)-1H-pyrazolo[3,4-b]pyridin-3-carbonitril, mit 2-Amino-pyridincarbonitril umgesetzt. Anschließend wurde weitere 3 h bei 180° C unter Mikrowellenbestrahlung erhitzt. Schließlich wurden weitere 80 mg (0.67 mmol) 2-Amino-pyridincarbonitril und 73 mg (0.65 mmol) Kalium-tert.-butylat zugegeben und 3 h bei 180°C unter Mikrowellenbestrahlung erhitzt.
Ausbeute: 6.6 mg (2 % d. Th.)
LC-MS (Methode 1): Rₜ = 0.89 min; MS (EIpos): m/z = 428 [M+H]⁺.
¹H-NMR (400MHz, DMSO-d₆): δ [ppm]= 2.94 - 3.12 (m, 2H), 4.93 (t, 2H), 7.50 - 7.59 (m, 1H), 8.37 (br. s., 1H), 8.70 (d, 1H), 8.75 (br. s., 1H), 8.91 (dd, 1H), 9.04 (br. s., 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., *Anal. Biochem.* 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| **Beispiel** | **MEC [µM]** |
|---|---|
| 1 | 0.3 |
| 2 | 0.1 |
| 3 | 10 |
| 4 | 0.3 |
| 5 | 0.3 |
| 7 | 0.01 |
| 8 | 0.1 |
| 9 | 0.03 |
| 10 | 0.3 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### C. Ausführunssbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindungen der folgenden Formeln
6- {5-Fluor-1-[(3-fluorpyridin-2-yl)methyl]-1H-pyrazolo[3,4-b]pyridin-3-yl}-3-methyl-1H-pyrazolo[3,4-d]pyrimidin-4-amin 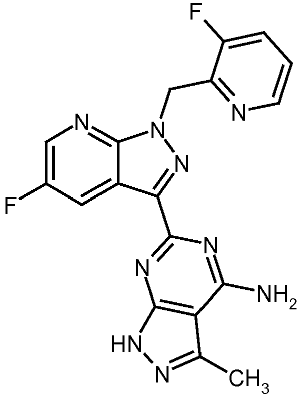
N³,N³-Diethyl-6-{5-fluor-1-[(3-fluorpyridin-2-yl)methyl]-1H-pyrazolo[3,4-b]pyridin-3-yl}-1H-pyrazolo[3,4-d]pyrimidin-3,4-diamin 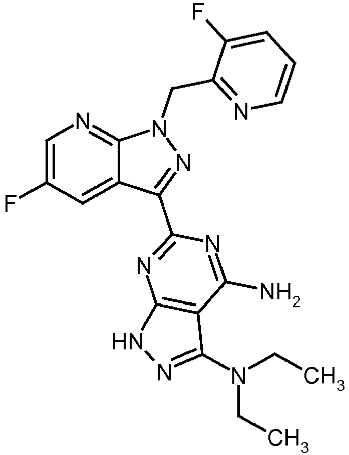
2-{5-Fluor-1-[(3-fluorpyridin-2-yl)methyl]-1H-pyrazolo[3,4-b]pyridin-3-yl}pyrido[2,3-d]pyrimidin-4-amin 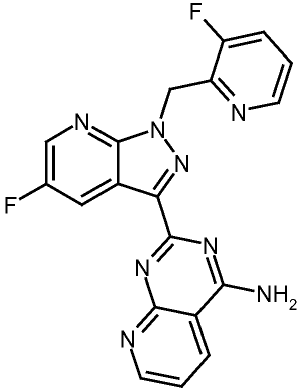
6-{5-Fluor-1-[(3-fluorpyridin-2-yl)methyl]-1H-pyrazolo[3,4-b]pyridin-3-yl}-3-methoxy-1H-pyrazolo[3,4-d]pyrimidin-4-amin 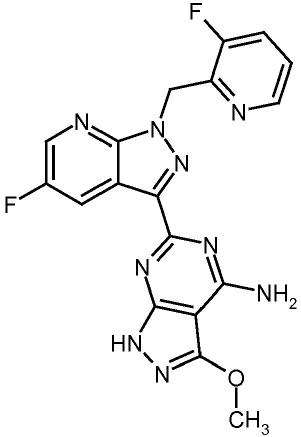
6-[(Cyclopropylmethyl)amino]-2-[5-fluor-1-(2-fluorbenzyl)-1H-pyrazolo[3,4-b]pyridin-3-yl]-7-(2,2,2-trifluorethyl)-7,9-dihydro-8H-purin-8-on 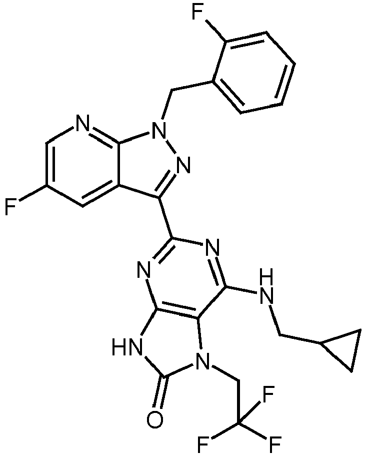
2-[5-Fluor-1-(2-fluorbenzyl)-1H-pyrazolo[3,4-b]pyridin-3-yl]-7-(2,2,2-trifluorethyl)-6-[(3,3,3-trifluorpropyl)amino]-7,9-dihydro-8H-purin-8-on 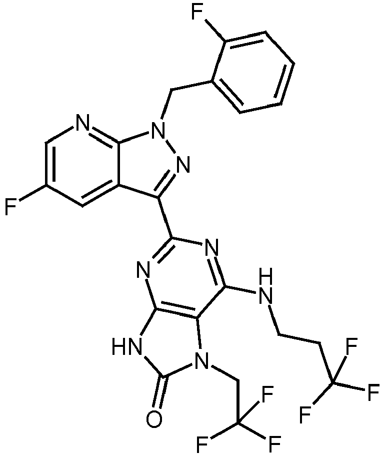
6-[7-(2-Fluorbenzyl)imidazo[1,5-b]pyridazin-5-yl]-3-methyl-1H-pyrazolo[3,4-d]pyrimidin-4-amin 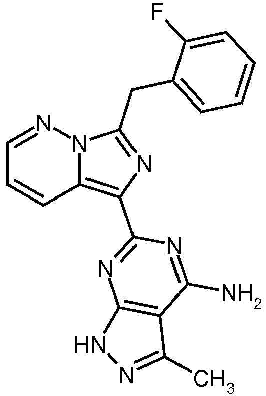
6-[5-Fluor-1-(3,3,4,4,4-pentafluorbutyl)-1H-pyrazolo[3,4-b]pyridin-3-yl]-3-methyl-1H-pyrazolo[3,4-d]pyrimidin-4-amin 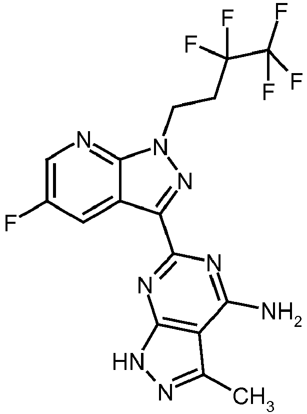
N3,N3-Diethyl-6-{5-fluor-1-[(3-fluorpyridin-2-yl)methyl]-1H-pyrazolo[3,4-b]pyridin-3-yl}-1H-pyrazolo[3,4-d]pyrimidin-3,4-diamin 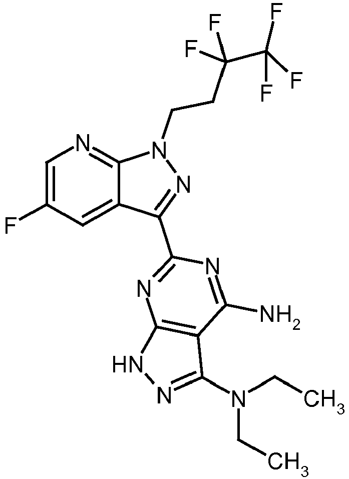
2-[5-Fluor-1-(3,3,4,4,4-pentafluorbutyl)-1H-pyrazolo[3,4-b]pyridin-3-yl]pyrido[2,3-d]pyrimidin-4-amin

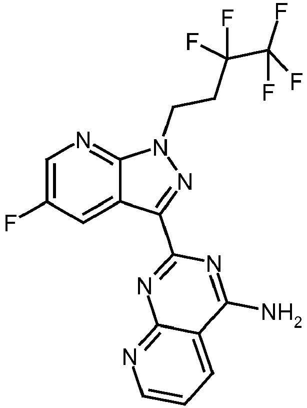

2. Verbindung der Formeln, wie in Anspruch 1 definiert, zur Verwendung bei der Behandlung und/oder Prophylaxe von Krankheiten.

3. Verbindung der Formeln, wie in Anspruch 1 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

4. Arzneimittel enthaltend eine Verbindung der Formeln, wie in Anspruch 1 definiert, in Kombination mit einem inerten, nicht-toxischen, pharmazeutisch geeigneten Hilfsstoff.

5. Arzneimittel enthaltend eine Verbindung der Formeln, wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

6. Arzneimittel nach Anspruch 4 oder 5 zur Verwendung bei der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compounds of the following formulae
6-{5-fluoro-1-[(3-fluoropyridin-2-yl)methyl]-1H-pyrazolo[3,4-b]pyridin-3-yl}-3-methyl-1H-pyrazolo[3,4-d]pyrimidine-4-amine 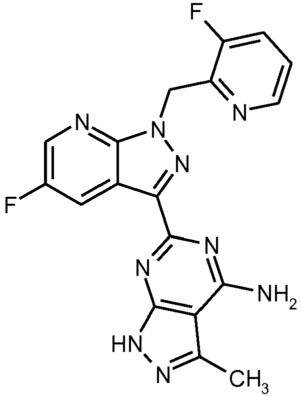
N³,N³-diethyl-6-{5-fluoro-1-[(3-fluoropyridin-2-yl)methyl]-1H-pyrazolo[3,4-b]pyridin-3-yl}-1H-pyrazolo[3,4-d]pyrimidine-3,4-diamine 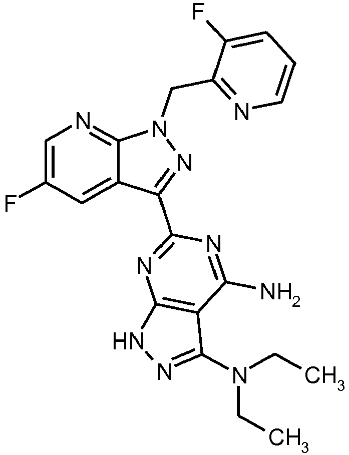
2-{5-fluoro-1-[(3-fluoropyridin-2-yl)methyl]-1H-pyrazolo[3,4-b]pyridin-3-yl}pyrido[2,3-d]pyrimidine-4-amine 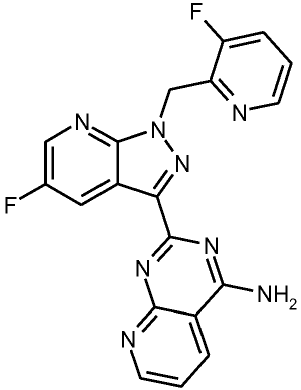
6-{5-fluoro-1-[(3-fluoropyridin-2-yl)methyl]-1H-pyrazolo[3,4-b]pyridin-3-yl}-3-methoxy-1H-pyrazolo[3,4-d]pyrimidine-4-amine 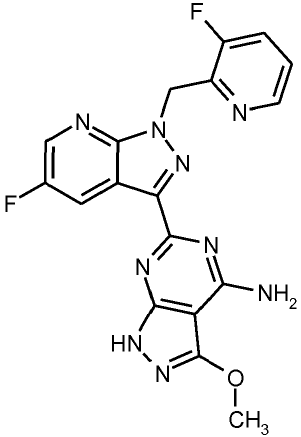
6-[(cyclopropylmethyl)amino]-2-[5-fluoro1-(2-fluorobenzyl)-1H-pyrazolo[3,4-b]pyridin-3-yl]-7-(2,2,2-trifluoroethyl)-7,9-dihydro-8H-purin-8-one 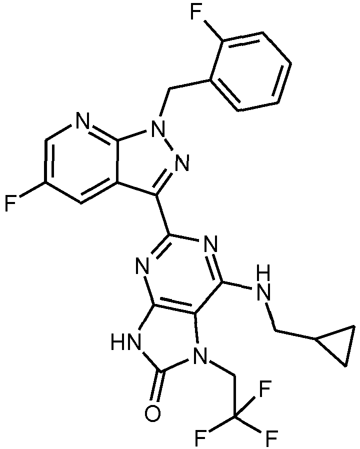
2-[5-fluoro-1-(2-fluorobenzyl)-1H-pyrazolo[3,4-b]pyridin-3-yl]-7-(2,2,2-trifluoroethyl)-6-[(3,3,3-trifluoropropyl)amino]-7,9-dihydro-8H-purin-8-one 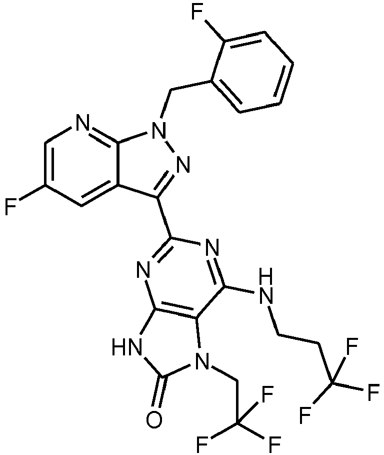
6-[7-(2-fluorobenzyl)imidazo[1,5-b]pyridazin-5-yl]-3-methyl-1H-pyrazolo[3,4-d]pyrimidine-4-amine 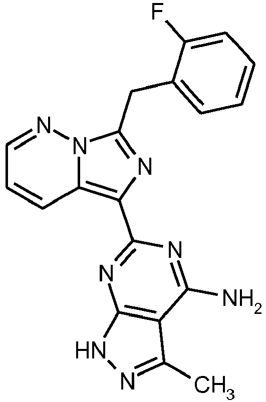
6-[5-fluoro-1-(3,3,4,4,4-pentafluorobutyl)-1H-pyrazolo[3,4-b]pyridin-3-yl]-3-methyl-1H-pyrazolo[3,4-d]pyrimidine-4-amine 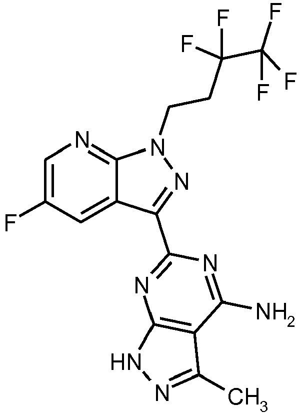
N3,N3-diethyl-6-{5-fluoro-1-[(3-fluoropyridin-2-yl)methyl]-1H-pyrazolo[3,4-b]pyridin-3-yl}-1H-pyrazolo[3,4-d]pyrimidine-3,4-diamine 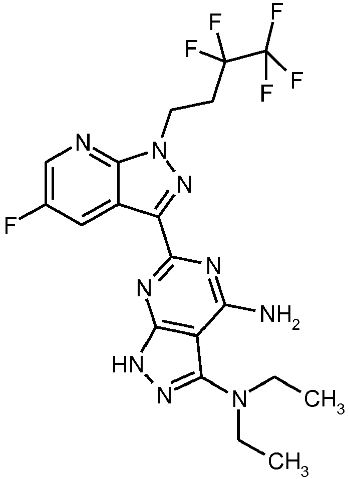
2-[5-fluoro-1-(3,3,4,4,4-pentafluorobutyl)-1H-pyrazolo[3,4-b]pyridin-3-yl]pyrido[2,3-d]pyrimidine-4-amine

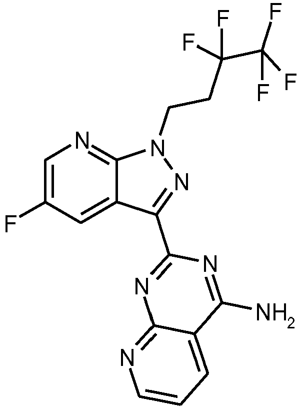

2. Compound of the formulae as defined in Claim 1 for use in the treatment and/or prophylaxis of diseases.

3. Compound of the formulae as defined in Claim 1 for use in a method for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

4. Medicament comprising a compound of the formulae as defined in Claim 1 in combination with an inert, non-toxic, pharmaceutically suitable excipient.

5. Medicament comprising a compound of the formulae as defined in Claim 1 in combination with a further active compound selected from the group consisting of organic nitrates, NO donors, cGMP-PDE inhibitors, antithrombotic agents, hypotensive agents and lipid metabolism modifiers.

6. Medicament according to Claim 4 or 5 for use in the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

## Revendications

1. Composés des formules suivantes :
6-{5-fluoro-1-[(3-fluoropyridin-2-yl)méthyl]-1H-pyrazolo[3,4-b]pyridin-3-yl}-3-méthyl-1H-pyrazolo[3,4-d]pyrimidin-4-amine 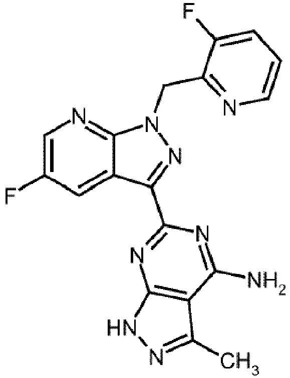
N³,N³-diéthyl-6-{5-fluoro-1-[(3-fluoropyridin-2-yl)méthyl]-1H-pyrazolo[3,4-b]pyridin-3-yl}-1H-pyrazolo[3,4-d]pyrimidin-3,4-diamine 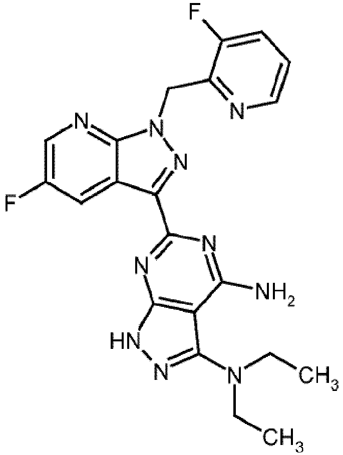
2-{5-fluoro-1-[(3-fluoropyridin-2-yl)méthyl]-1H-pyrazolo[3,4-b]pyridin-3-yl}pyrido[2,3-d]pyrimidin-4-amine 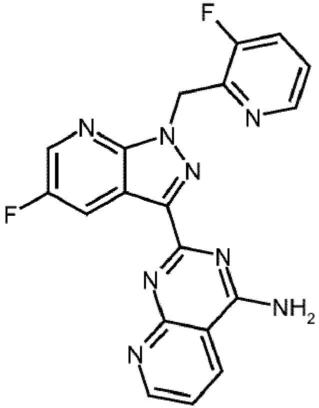
6-{5-fluoro-1-[(3-fluoropyridin-2-yl)méthyl]-1H-pyrazolo[3,4-b]pyridin-3-yl}-3-méthoxy-1H-pyrazolo[3,4-d]pyrimidin-4-amine 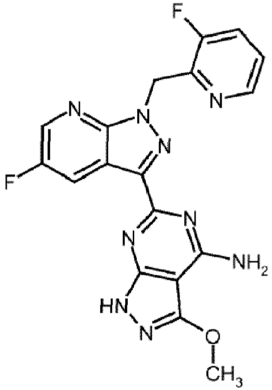
6-[(cyclopropylméthyl)amino]-2-[5-fluoro-1-(2-fluorobenzyl)-1H-pyrazolo[3,4-b]pyridin-3-yl]-7-(2,2,2-trifluoroéthyl)-7,9-dihydro-8H-purin-8-one 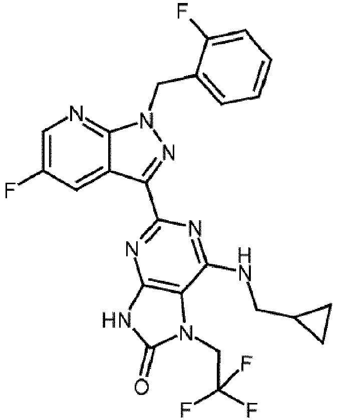
2-[5-fluoro-1-(2-fluorobenzyl)-1H-pyrazolo[3,4-b]pyridin-3-yl]-7-(2,2,2-trifluoroéthyl)-6-[(3,3,3-trifluoropropyl)amino]-7,9-dihydro-8H-purin-8-one 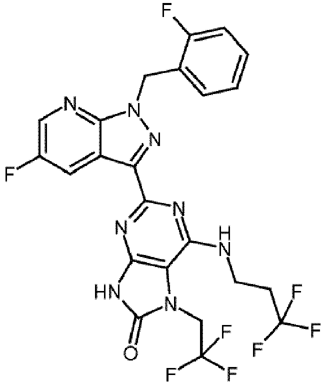
6-[7-(2-fluorobenzyl)imidazo[1,5-b]pyridazin-5-yl]-3-méthyl-1H-pyrazolo[3,4-d]pyrimidin-4-amine 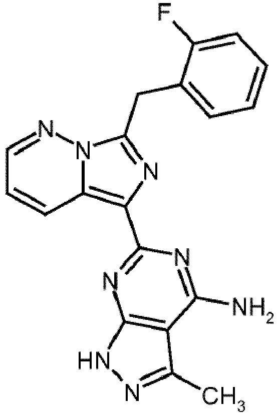
6-[5-fluoro-1-(3,3,4,4,4-pentafluorobutyl)-1H-pyrazolo[3,4-b]pyridin-3-yl]-3-méthyl-1H-pyrazolo[3,4-d]pyrimidin-4-amine 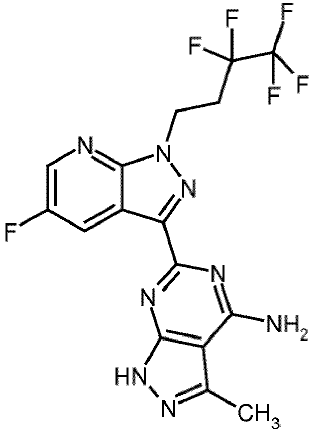
N3,N3-diéthyl-6-{5-fluoro-1-[(3-fluoropyridin-2-yl)méthyl]-1H-pyrazolo[3,4-b]pyridin-3-yl}-1H-pyrazolo[3,4-d]pyrimidin-3,4-diamine 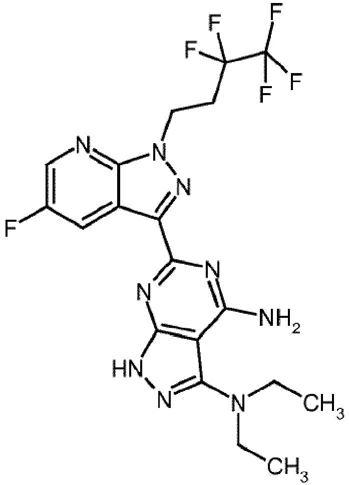
2-[5-fluoro-1-(3,3,4,4,4-pentafluorobutyl)-1H-pyrazolo[3,4-b]pyridin-3-yl]pyrido[2,3-d]pyrimidin-4-amine

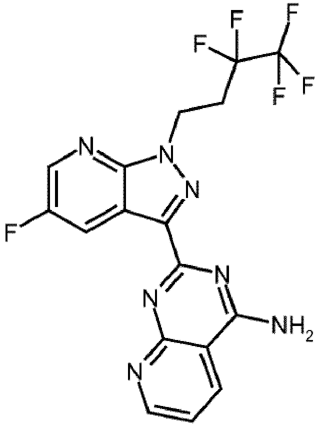

2. Composé des formules telles que définies dans la revendication 1, destiné à une utilisation pour le traitement et/ou la prophylaxie de maladies.

3. Composé des formules telles que définies dans la revendication 1, destiné à une utilisation dans un procédé de traitement et/ou de prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

4. Médicament contenant un composé des formules telles que définies dans la revendication 1, en combinaison avec un adjuvant inerte, non toxique, pharmaceutiquement approprié.

5. Médicament contenant un composé des formules telles que définies dans la revendication 1, en combinaison avec un autre agent actif choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

6. Médicament selon la revendication 4 ou 5, destiné à une utilisation pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.
